# EUROPEAN PATENT APPLICATION

(11) **EP 4 265 270 A1**
(43) Date of publication of application: **25.10.2023**
(21) Application number: 21906683.4
(22) Date of filing: 16.12.2021
(51) Int. Cl.: A61K 38/46, A61K 35/30, A61K 38/02, A61K 38/17, A61K 38/19, A61K 38/47, A61K 38/48, A61K 39/385, A61K 48/00, A61L 27/18, A61L 27/22, A61L 27/38, A61L 27/40, A61L 27/54, A61P 9/10, A61P 21/00, A61P 25/00, A61P 25/02, A61P 25/08, A61P 25/14, A61P 25/16

(54) **NEURON ACTIVATOR**

(30) Priority: 17.12.2020 JP 2020209492; 17.12.2020 JP 2020209568
(71) Applicant: Kyoto University, Kyoto-shi, Kyoto 606-8501 (JP)
(72) Inventor: TAKAHASHI, Jun, Kyoto-shi Kyoto 606-8501 (JP); SAMATA, Bumpei, Kyoto-shi Kyoto 606-8501 (JP)
(74) Representative: Vossius & Partner Patentanwälte Rechtsanwälte mbB
(86) International application number: PCT/JP2021/046462
(87) International publication number: WO 2022/131322

(57) **Abstract**

An object of the invention is to develop and provide an agent for activating a neuron which activates a brain neuron. Another object of the invention is to provide an agent for promoting at least one of, in cell transplantation for a neural disease, survival of a transplanted cell, differentiation of the transplanted cell into a neurononal cell, and maturation of the neuron. Provided are an agent for activating neuron comprising a protein selected from the group consisting of Lysozyme (LYZ), Secreted phosphoprotein 1 (SPP1), Progranulin (PGrn), Cathepsin D (Ctsd), Cathepsin S (Ctss), Apolipoprotein D (Apod), Sparc, CD52, Macrophage expressed gene 1 (MPEG1), C-C motif chemokine ligand 2 (CCL2), Cystatin F (CST7), Ras family small GTPase 2 (RAC2), Serpin family A member 3 (SERPINA3), Anosmin 1 (ANOS 1), Kallikrein-related peptidase 6 (KLK6), Alpha-2-macroglobulin (A2M), GM14295, Galectin 3-binding protein (LGALS3BP), Apolipoprotein C1 (APOC1), and orthologs thereof, or an active fragment thereof, or a nucleic acid encoding the protein or active fragment thereof, and an agent for assisting cell transplantation comprising said agent.

## Description

### Technical Field

The present invention relates to an agent for activating a neuron, an agent for assisting cell transplantation, an agent for treating or preventing a neural disease, a composition for treating a neural disease, and the like.

### Background Art

In the adult brain, it is difficult for neurons that have been once lost to spontaneously regenerate. Therefore, if a neurodegenerative disease, cerebral infarction, head trauma, or other causes leads to loss of brain neurons and then loss of brain functions, spontaneous restoration of the brain functions is very difficult.

As an approach for prevention or treatment of neural diseases associated with degeneration or loss of neurons, a method for reducing further cell deaths of neurons has been attempted. Such method is primarily intended to delay the development or progression of neural disease, and it is difficult to restore the brain function that has been once lost.

Meanwhile, a method for supplementing neurons by transplantation is also performed for the purpose of recovery of brain functions. To date, several hundred cases of transplantation therapy using human neurons have been performed. An example thereof includes human fetal ventral mesencephalic tissue transplantation for patients of Parkinson's disease, which has been performed since the1980s.

In general, however, the survival rate of transplanted cells is low in transplantation therapy of neural diseases, and the proportion of cells that can differentiate into neurons and sufficiently mature is very low, which have been major obstacles for therapeutic effects.

### Prior Art Literature

### Non-patent Literatures

Non-patent Literature 1: Peron S., et al., J. Neurosci., 2017, 37 (7): 1820-1834

### Summary of the Invention

### Problems to Be Solved by the Invention

An object of the present invention is to develop and provide an agent for activating a neuron which activates a brain neuron. Another object of the present invention is to provide an agent for promoting at least one of survival of transplanted cells, differentiation of the survived cells into neurons, and maturation of the neurons, in cell transplantation for a neural disease.

### Means for Solving the Problems

Under the technical background as described above, the present inventors have intensively conducted studies, and as a result, found that Cst7, Spp1, A2m, Apoc1, Ccl12, Lyz2, Klk6, Gm11428, Cd52, Lgals3bp, Mpeg1, Rac2, Serpina3n, Gm14295, and the like have an effect of activating a neuron, such as an effect of promoting extension of an axon of a neuron, an effect of increasing a neural axon collateral, an effect of suppressing cell death of a neuron, and an effect of increasing the number of neurons. The present inventors further found that, in cell transplantation of a ventral mesencephalic tissue into the striatum of mouse, Lyz2 considerably increases the density of the dopaminergic neurons derived from the transplanted cells to a significant extent, and completed the present invention. The present invention is based on the findings and provides the following.

(1) An agent for activating a neuron, comprising a protein or an active fragment thereof, or a nucleic acid encoding the protein or active fragment thereof,
   wherein the protein is selected from the group consisting of Lysozyme (LYZ), Secreted phosphoprotein 1 (SPP1), Progranulin (PGrn), Cathepsin D (Ctsd), Cathepsin S (Ctss), Apolipoprotein D (Apod), Sparc, CD52, Macrophage expressed gene 1 (MPEG1), C-C motif chemokine ligand 2 (CCL2), Cystatin F (CST7), Ras family small GTPase 2 (RAC2), Serpin family A member 3 (SERPINA3), Anosmin 1 (ANOS1), Kallikrein-related peptidase 6 (KLK6), Aalpha-2-macroglobulin (A2M), GM14295, Galectin 3-binding protein (LGALS3BP), Apolipoprotein C1 (APOC1), and orthologs thereof.
(2) The agent according to (1), wherein the neuron is a brain neuron.
(3) The agent according to (1) or (2), wherein said activating a neuron is a promotion of extension of an axon, an axon collateral, and/or a dendrite.
(4) The agent according to (1) or (2), wherein said activating a neuron is a suppression of neuronal degeneration and/or neuronal cell death.
(5) An agent for assisting cell transplantation comprising the agent according to any of (1) to (4).
(6) An agent for treating or preventing a neural disease comprising the agent according to any of (1) to (4).
(7) The agent according to (6), wherein the disease is selected from the group consisting of neurodegenerative disease, cerebral tumor, multiple sclerosis, epilepsia, head trauma, cerebral infarction, cerebral stroke, and ischemia.
(8) The agent according to (7), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
(9) A composition for treating or preventing a neural disease, comprising the agent according to any of (6) to (8).
(10) A method for activating a brain neuron, comprising a step of administering the agent according to any of (1) to (4) to a subject.
(11) A method for treating or preventing a neural disease in a subject, comprising a step of administering the agent according to any of (6) to (8) or the composition according to (9) to a subject.
(12) The method according to (11), wherein the disease is selected from the group consisting of neurodegenerative disease, cerebral tumor, multiple sclerosis, epilepsia, head trauma, cerebral infarction, cerebral stroke, and ischemia.
(13) The method according to (12), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
(14) The agent according to any of (1) to (4) for use in the treatment and/or prevention of a neural disease.
(15) The agent according to (14), wherein the disease is selected from the group consisting of neurodegenerative disease, cerebral tumor, multiple sclerosis, epilepsia, head trauma, cerebral infarction, cerebral stroke, and ischemia.
(16) The agent according to (15), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
(17) Use of the agent according to any of (1) to (4) in the manufacture of a medicine for treatment and/or prevention of a neural disease.
(18) The use according to (17), wherein the disease is selected from the group consisting of neurodegenerative disease, cerebral tumor, multiple sclerosis, epilepsia, head trauma, cerebral infarction, cerebral stroke, and ischemia.
(19) The use according to (18), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.

(1') An agent for assisting cell transplantation comprising a protein set forth below or a nucleic acid encoding said protein, wherein said protein is selected from the group consisting of Lysozyme (LYZ), Secreted phosphoprotein 1 (SPP1), Progranulin (PGrn), Cathepsin D (Ctsd), Cathepsin S (Ctss), Apolipoprotein D (Apod), Sparc, CD52, Macrophage expressed gene 1 (MPEG1), C-C motif chemokine ligand 2 (CCL2), cystatin F (CST7), Ras family small GTPase 2 (RAC2), Serpin family A member 3 (SERPINA3), Anosmin 1 (ANOS1), Kallikrein-related peptidase 6 (KLK6), Alpha-2-macroglobulin (A2M), GM14295, Galectin 3-binding protein (LGALS3BP), Apolipoprotein C1 (APOC1), and orthologs thereof.
(2') The agent for assisting cell transplantation according to (1'), wherein the cell is one or more cells selected from the group consisting of a neural stem cell, a neural progenitor cell, and a neuron.
(3') The agent for assisting cell transplantation according to (2'), wherein the neural stem cell, the neural progenitor cell, and the neuron are a cell which is derived from a pluripotent stem cell through induced differentiation.
(4') The agent for assisting cell transplantation according to any of (1') to (3'), which is administered simultaneously with the cell transplantation or after the transplantation to a recipient.
(5') The agent for assisting cell transplantation according to any of (1') to (4'), which is used in a mixture with a transplanted cell.
(6') The agent for assisting cell transplantation according to any of (1') to (5') for use in the treatment of a neural disease.
(7') The agent for assisting cell transplantation according to (6'), wherein the disease is selected from the group consisting of neurodegenerative disease, cerebral tumor, multiple sclerosis, epilepsia, head trauma, cerebral infarction, cerebral stroke, and ischemia.
(8') The agent for assisting cell transplantation according to (7'), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
(9') A composition for treating a neural disease, comprising the agent for cell transplantation according to any of (1') to (8') and a cell for transplantation.
(10') A method for transplanting a cell into a recipient, comprising a step of administering an agent for assisting cell transplantation in which the agent for cell transplantation according to (1') is administered to the recipient, and a step of cell transplantation in which the cell is transplanted to the recipient.
(11') The method according to (10'), wherein the cell is one or more cells selected from the group consisting of a neural stem cell, a neural progenitor cell, and a neuron.
(12') The method according to (11'), wherein the neural stem cell, the neural progenitor cell, and the neuron are a cell which are derived from a pluripotent stem cell through induced differentiation.
(13') The method according to any of (10') to (12'), wherein the step of administering an agent for assisting cell transplantation is performed simultaneously with or after the step of cell transplantation.
(14') The method according to any of (10') to (13'), wherein the agent for assisting cell transplantation is administered in a mixture with a transplanted cell.
(15') A method for treating a neural disease in a recipient, comprising a step of administering an agent for assisting cell transplantation in which the agent for cell transplantation according to (1') is administered to the recipient, and a step of cell transplantation in which the cell is transplanted to the recipient.
(16') The method according to (15'), wherein the neural disease is selected from the group consisting of neurodegenerative disease, cerebral tumor, multiple sclerosis, epilepsia, head trauma, cerebral infarction, cerebral stroke, and ischemia.
(17') The method according to (16'), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
(18') The agent for assisting cell transplantation according to any of (1') to (5') for use in the treatment of a neural disease.
(19') The agent for assisting cell transplantation according to (18'), wherein the neural disease is selected from the group consisting of neurodegenerative disease, cerebral tumor, multiple sclerosis, epilepsia, head trauma, cerebral infarction, cerebral stroke, and ischemia.
(20') The agent for assisting cell transplantation according to (19'), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
(21') Use of the agent for assisting cell transplantation according to any of (1') to (5') in the manufacture of a medicine for the treatment of a neural disease.
(22') The use according to (21'), wherein the neural disease is selected from the group consisting of neurodegenerative disease, cerebral tumor, multiple sclerosis, epilepsia, head trauma, cerebral infarction, cerebral stroke, and ischemia.
(23') The use according to (22'), wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.

The present specification encompasses the contents disclosed in Japanese Patent Application Nos. 2020-209492 and 2020-209568, which form the basis of the priority of the present application.

### Effects of the Invention

According to the agent for activating a neuron of the present invention, a brain neuron can be activated. Additionally, the agent for assisting cell transplantation of the present invention can provide an effect of promoting at least one of the number, the density, and the maturation degree of the neuron derived from the transplanted cell. Thus, an improved effect of transplantation is expected.

### Brief Description of the Drawings

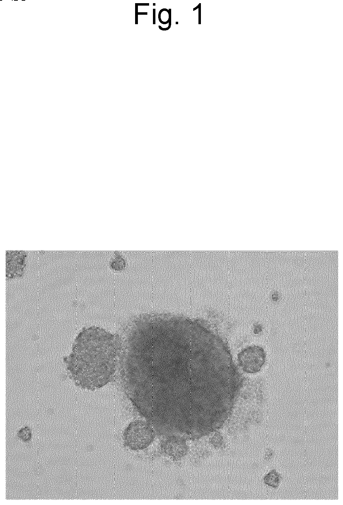

Figure 1 shows a cell aggregate prepared from the cerebral cortex tissue of a mouse on embryonic day 13.5.

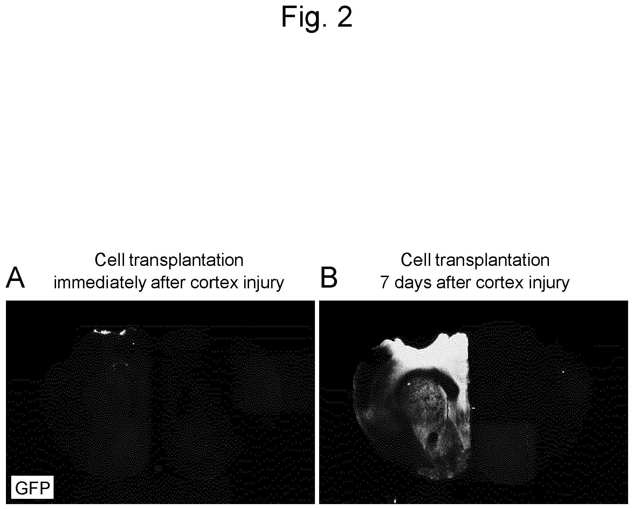

Figure 2 shows the results of transplantation of a cell aggregate into a model of cortical injury. Fig. 2 A shows the results of transplantation of a cell aggregate immediately after cortical injury. Fig. 2 B shows the results of transplantation of a cell aggregate 7 days after cortical injury. Only donor-derived cells are stained with the anti-GFP antibody.

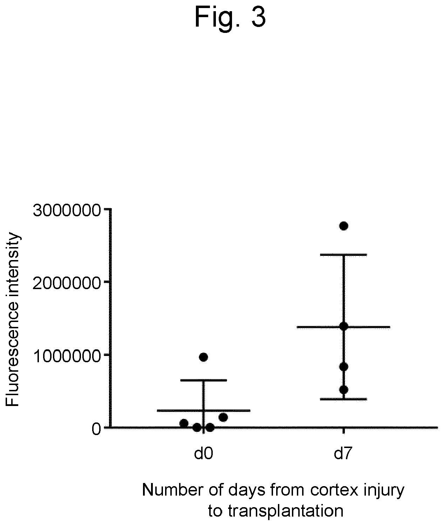

Figure 3 shows the results of quantification of the fluorescence intensity derived from the neuronal axon of the transplanted cells in the striatum after cell transplantation was performed immediately after cortical injury (d0) or 7 days after cortical injury (d7).

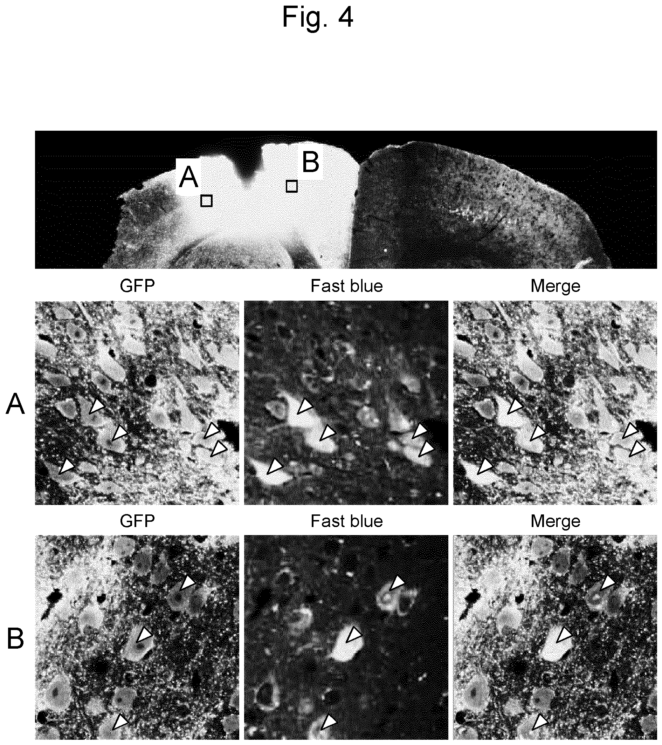

Figure 4 shows the results of retrograde labeling of the mouse subjected to cell transplantation 7 days after cortical injury with Fast blue through the first cervical nerve. Fig. 4 A and Fig. 4 B show enlarged diagrams of framed regions in the upper panel. An arrowhead indicates a GFP-positive and Fast blue-positive cell body in the transplant (*i.e*., a cell body of a neuron which is derived from the transplant and which projects an axon to the spinal cord).

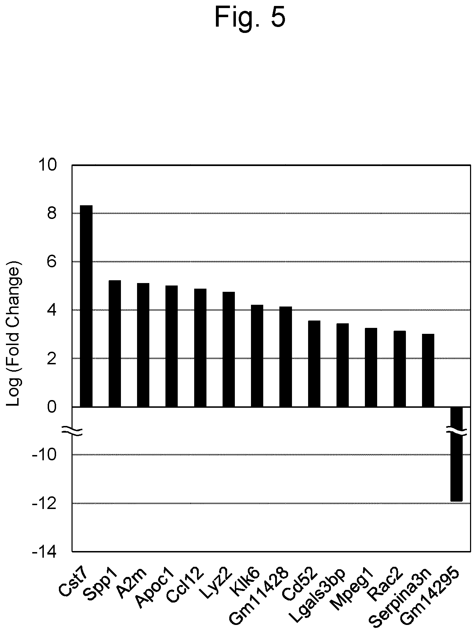

Figure 5 shows fold changes of gene expression 7 days after cortical injury in comparison with the gene expression immediately after cortical injury (Day 0) regarding each factor extracted as a gene whose expression level was increased or decreased 7 days after cortical injury.

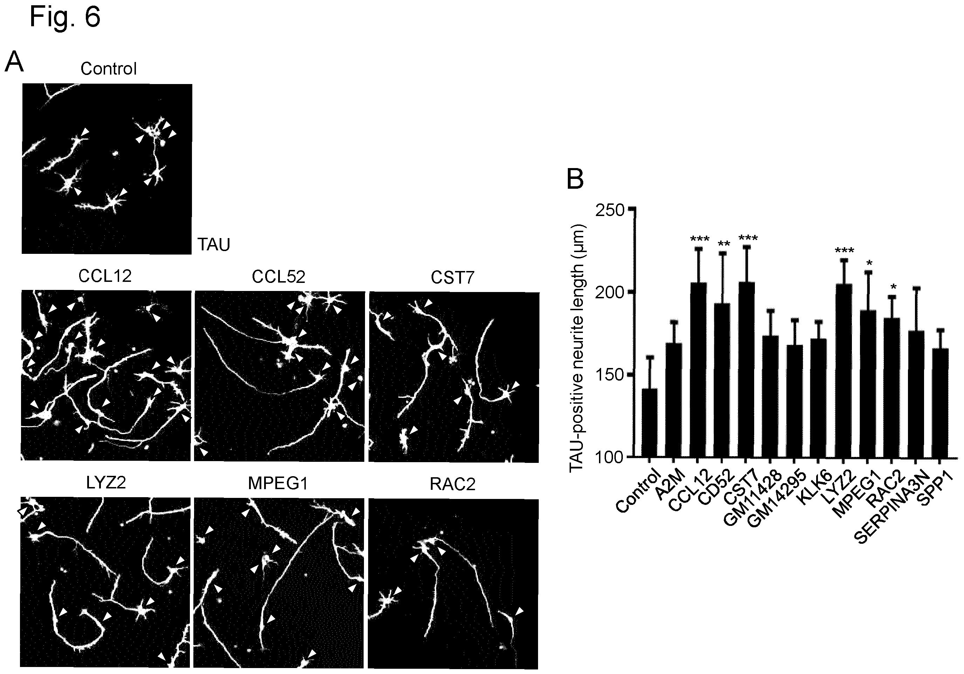

Figure 6 shows effects of promoting extension of neuronal axons for neurons derived from mouse cerebral cortex by the recombinant protein of each factor. Fig. 6 A shows neurons cultured in the presence of the recombinant protein of each factor. An arrowhead indicates the position of a cell body. Fig. 6 B shows the results of quantification of the length of TAU-positive neural axons. Error bars indicate the standard deviation, * indicates P < 0.05, ** indicates P < 0.01, and *** indicates P < 0.001 (one-way analysis of variance (one-way ANOVA)).

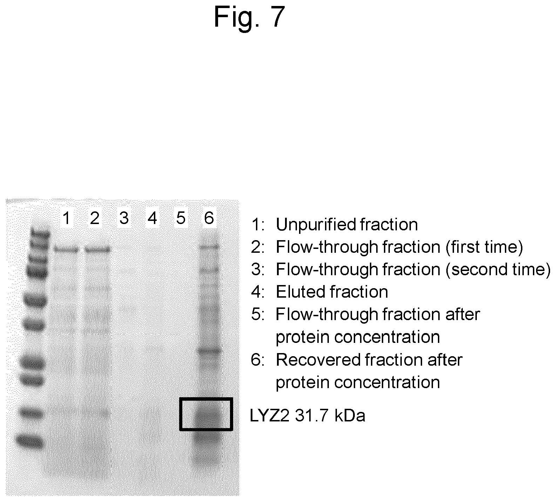

Figure 7 shows the results of detection by SDS-PAGE and CBB staining of the mouse LYZ22 (mLYZ2) recombinant protein that was expressed using the Brevibacillus expression system and subjected to His-tag purification and protein concentration.

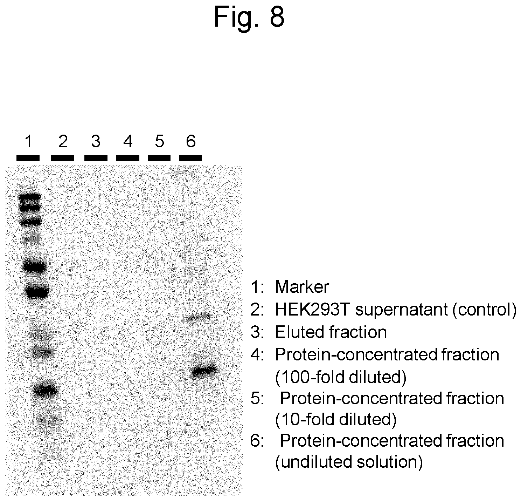

Figure 8 shows the results of detection by Western blotting of the mLYZ2 recombinant protein that was expressed using the Brevibacillus expression system and subjected to His-tag purification and protein concentration.

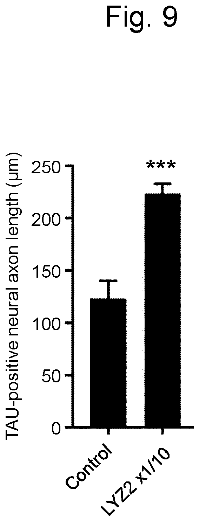

Figure 9 shows the activity of promoting the extention of neuronal axons by the mLYZ2 recombinant protein that was expressed using the Brevibacillus expression system and subjected to His-tag purification and protein concentration. Error bars indicate the standard deviation, and *** indicates p < 0.001 (Student's t test).

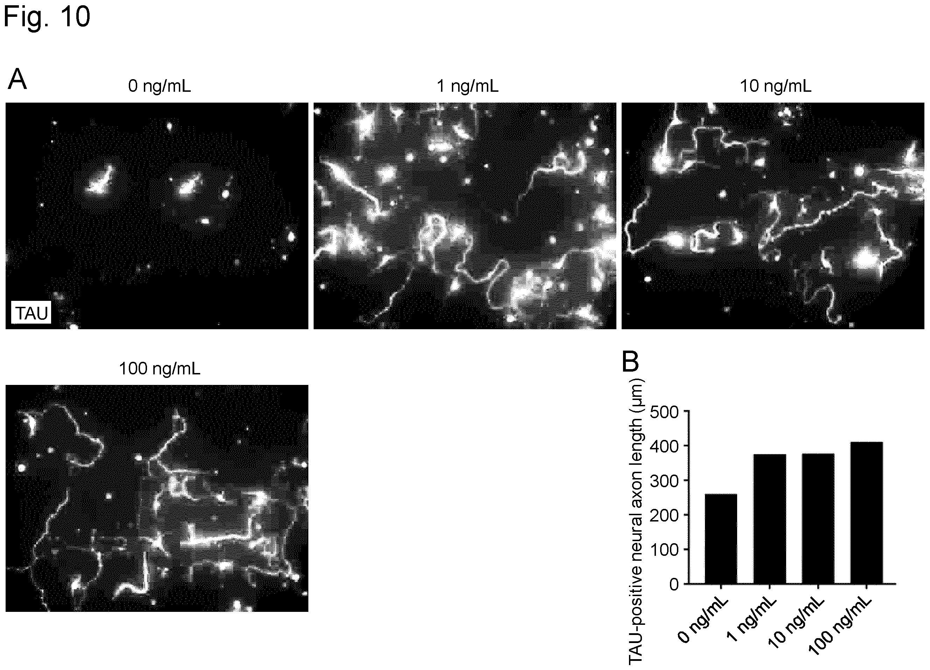

Figure 10 shows the results in which the extension of TAU-positive neuronal axons was promoted by a commercially available mLYZ2 recombinant protein. Fig. 10 A shows neurons cultured in the presence of the mLYZ2 recombinant proteins at each concentration. Fig. 10 B shows the results of quantification of the length of TAU-positive neuronal axons.

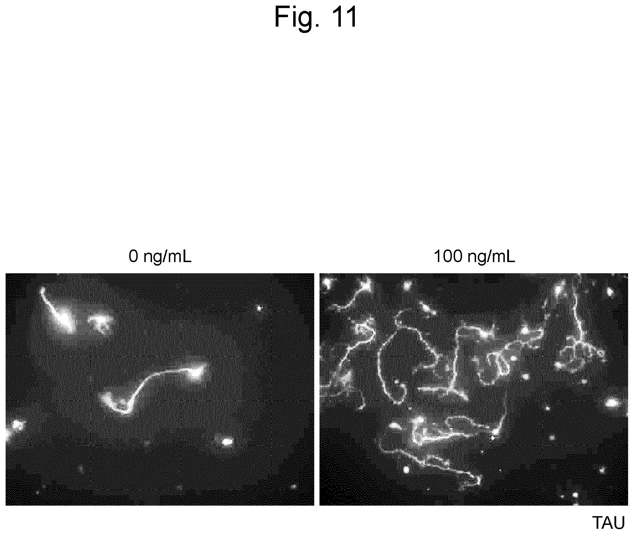

Figure 11 shows the results in which the number of neuronal axon collaterals of the TAU-positive neuronal axons were increased by a commercially available mLYZ2 recombinant protein.

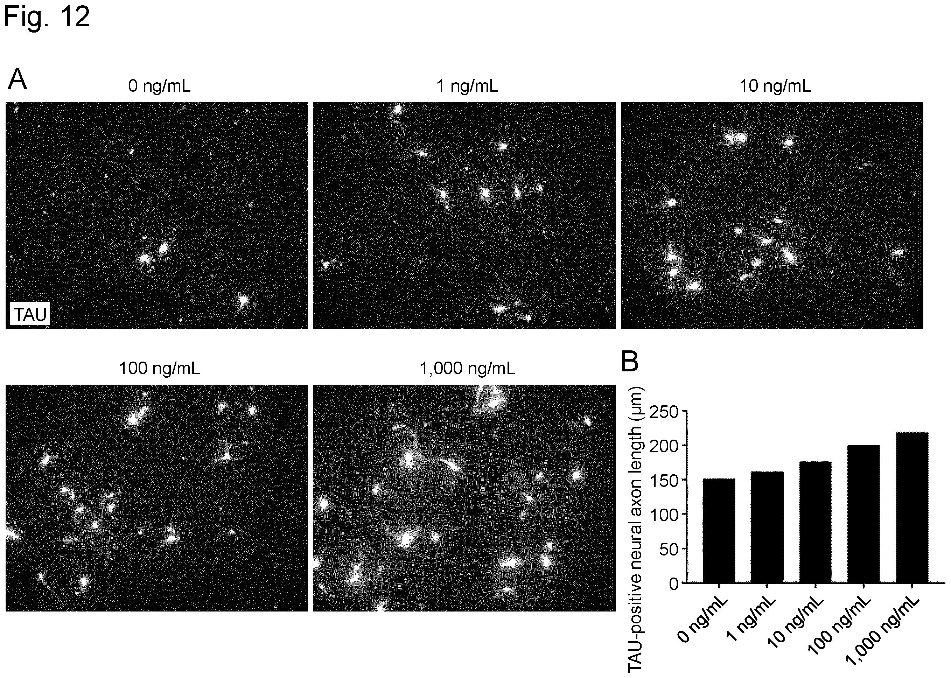

Figure 12 shows the results in which the extension of the TAU-positive neuronal axons of the neurons derived from the cerebral cortex organoid derived from human ES cells was promoted by a human LYZ (hLYZ). Fig. 12 A shows neurons cultured in the presence of hLYZ at each concentration. Fig. 12 B shows the results of quantification of the length of the TAU-positive neuronal axons.

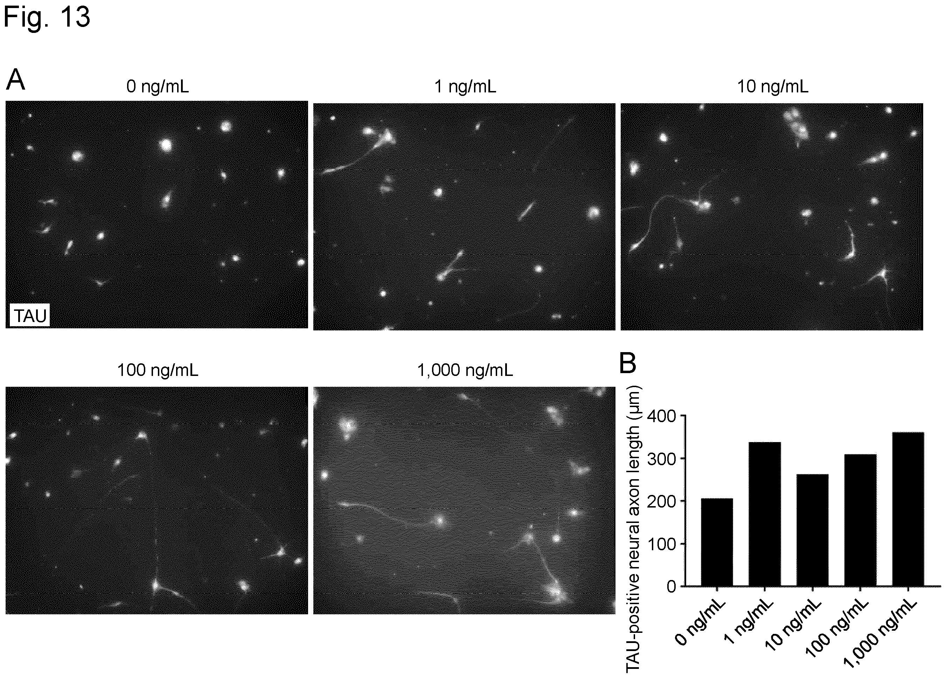

Figure 13 shows the results in which the extension of the TAU-positive neuronal axons of dopamine neurons derived from human ES cells was promoted by hLYZ. Fig. 13 A shows neurons cultured in the presence of hLYZ at each concentration. Fig. 13 B shows the results of quantification of the length of the TAU-positive neuronal axons.

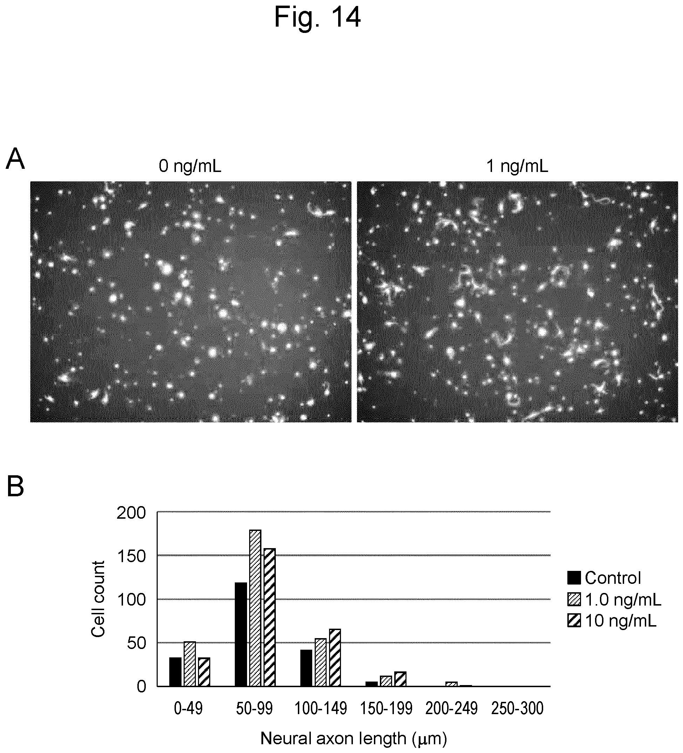

Figure 14 shows the results in which the total cell number of the TAU-positive neurons derived from the mouse cerebral cortex was increased by a commercially available mLYZ2 recombinant protein. Fig. 14 A shows neurons cultured in the presence or absence of mLYZ2. Fig. 14 B shows the results of quantification of the length of the TAU-positive neuronal axons.

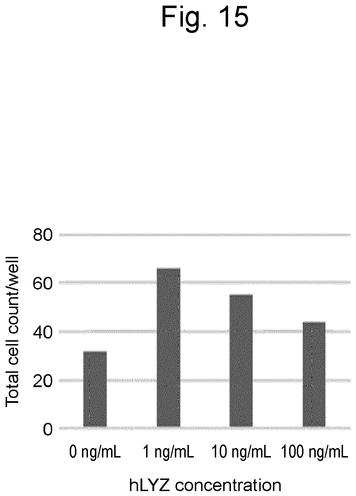

Figure 15 shows the results in which the total cell number of TAU-positive neurons derived from the cerebral cortex organoid derived from human iPS cells are increased by a commercially available hLYZ recombinant protein.

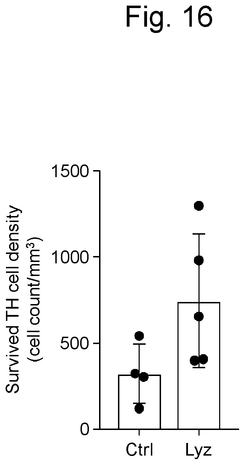

Figure 16 shows the results of analysis of the density of the TH-positive cells (dopamine-positive cells) in the transplant in the mouse one month after transplantation of the embryonic mouse ventral mesencephalic tissue. Fig. 16 shows the results of the group to which the mLYZ2 recombinant protein was administered simultaneously with cell transplantation (Lyz) and the group to which the mLYZ2 recombinant protein was not administered (Ctrl).

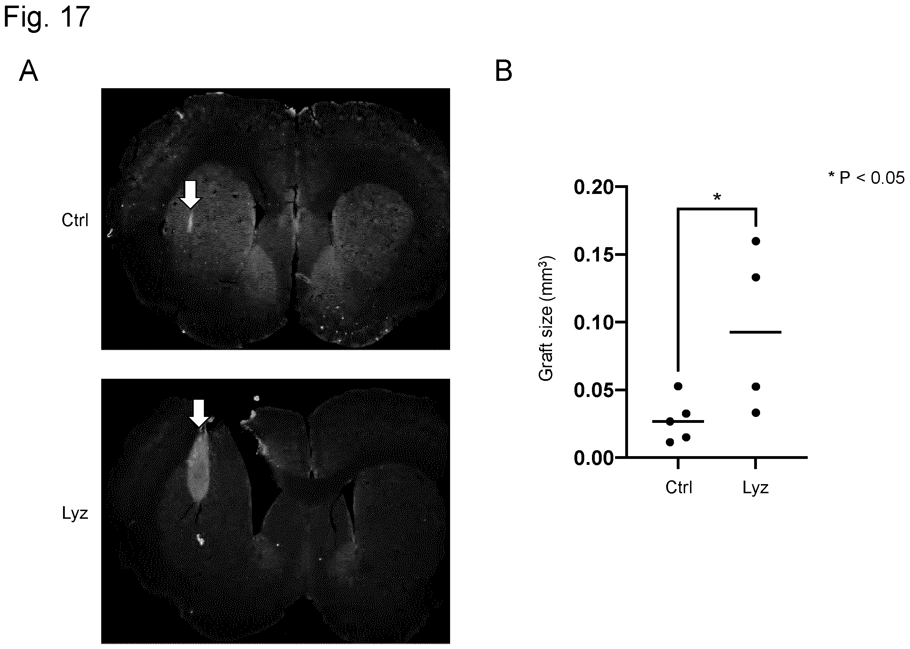

Figure 17 shows the results of analysis of the transplant size 3 months after transplantation in the SCID mouse in which dopamine neuron progenitor cells derived from the human ES cells were transplanted into the striatum. Fig. 17 shows the results of the group in which the human Lyz recombinant protein was added to the culture medium in the culture before cell transplantation (Lyz) and the group in which it was not added (Ctrl). In Fig. 17 A, the transplant of the Ctrl group and that of the Lyz group are indicated by arrows. Fig. 17 B shows the size of the transplant. A horizontal line of each group indicates a mean value. * indicates p < 0.05 (Student's t test).

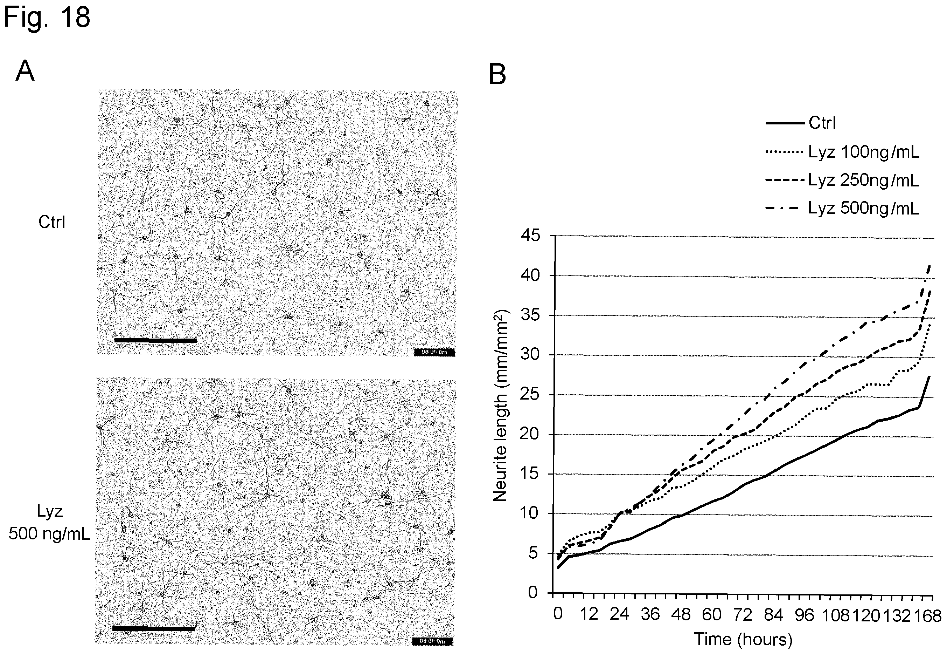

Figure 18 shows temporal changes of the length of axons when the cells dissociated from the cerebral cortex organoid derived from human iPS cells were cultured in the presence of the human LYZ recombinant protein (500 ng/mL). Fig. 18 A shows axons in the absence of LYZ (Ctrl) and in the presence of LYZ (Lyz 500 ng/mL). The scale bar at the bottom left in the photograph indicates 200 µm. Fig. 18 B shows temporal changes of length of axons on day 0 to day 8 (0 to 168 hours) of culture. The length of axons shown on the vertical axis indicates a total value (mm) of the length of the axons included in the unit area (mm²) of the obtained image.

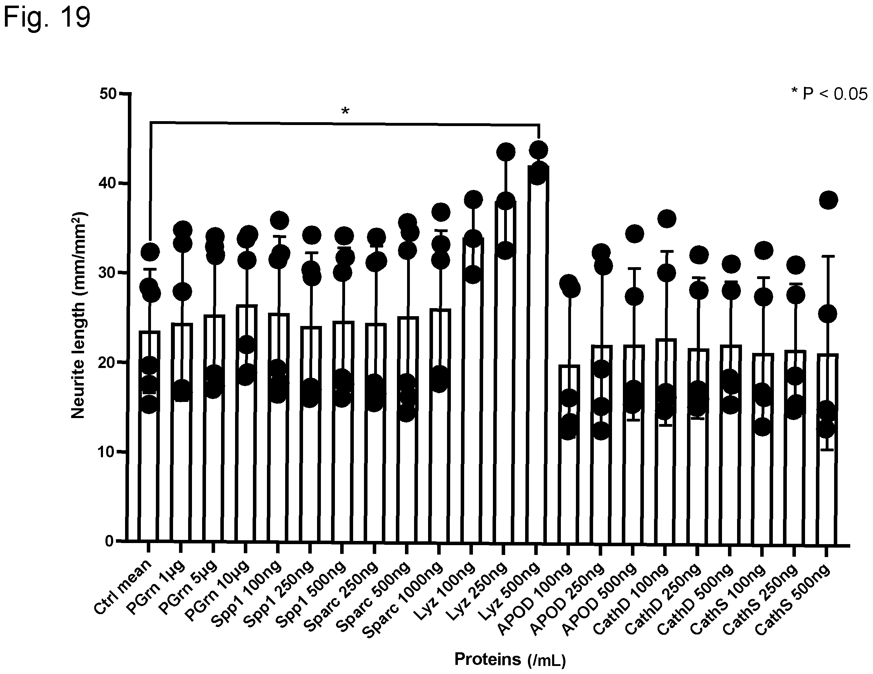

Figure 19 shows the results of quantification of the length of axons on day 7 of culture performed in the presence of each factor. In the figure, CathD indicates Cathepsin D (Ctsd), and CathS indicates Cathepsin S (Ctss). The amount of each factor shown in the figure indicates the amount in 1 mL of the medium. Error bars indicate a standard deviation, and * indicates P < 0.05 (One-way ANOVA with Holm-Sidak's post hoc analysis).

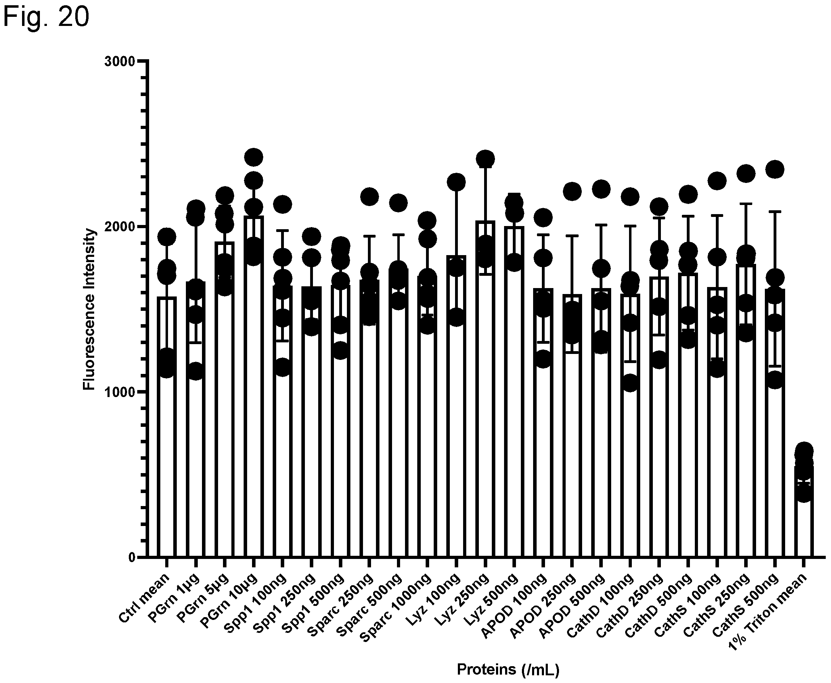

Figure 20 shows the results of evaluation of cell survival rate on day 7 of culture using Alamar Blue. In the figure, CathD indicates Cathepsin D (Ctsd), and CathS indicates Cathepsin S (Ctss). The amount of each factor shown in the figure indicates the amount in 1 mL of the medium. In the figure, "Ctrl mean" indicates a cell to which no candidate factor was added, and "1% Triton mean" indicates a cell that was artificially injured by surfactant (1% Triton) treatment. Error bars indicate a standard deviation.

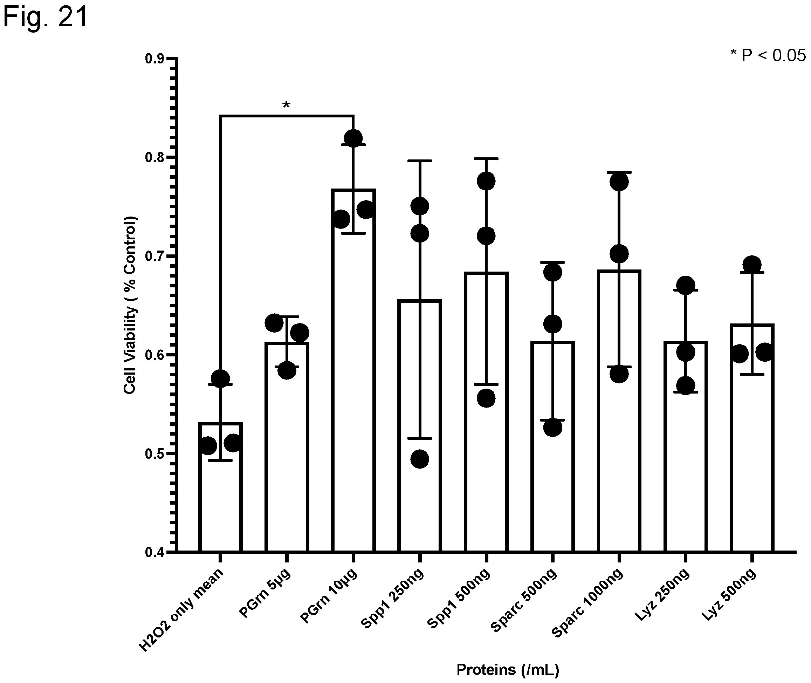

Figure 21 shows the results of evaluation of cell survival rate under cytotoxic (H₂O₂) conditions using Alamar Blue. In the figure, CathD indicates Cathepsin D (Ctsd), and CathS indicates Cathepsin S (Ctss). The amount of each factor shown in the figure indicates the amount in 1 mL of the medium. Error bars indicate standard errors, and * indicates P < 0.05 (One-way ANOVA with Holm-Sidak's post hoc analysis).

### Embodiments of the Invention

### 1. An agent for activating a neuron

### 1-1. Overview

The first aspect of the present invention is an agent for activating a neuron. The agent for activating a neuron of the present aspect comprises a protein selected from the group consisting of proteins such as LYZ, CD52, MPEG1, CCL2, CST7, RAC2, SERPINA3, ANOS1, KLK6, A2M, GM14295, SPP1, LGALS3BP, APOC1, PGrn, Ctsd, Ctss, Apod, and Sparc, an active fragment of any of the protein, and an ortholog of them or a nucleic acid encoding the protein. According to the agent for activating a neuron of the present invention, an effect of promoting the extension of axon and an effect of suppressing cell death for a brain neuron can be obtained.

### 1-2. Definitions

The term "activating a neuron (neuronal activation)" used herein refers to promotion of differentiation of a neural stem cell or a neural progenitor cell into a neuron, and an effect of promoting survival, differentiation, maturation, connection to other neurons, circuit formation, or a function of a neuron, or an effect of protecting survival, differentiation, maturation, connection to other neurons, circuit formation, or a function of a neuron against injury to the neuron. Examples of neuronal activation include, but are not limited to, promotion of extension and regeneration of a neurite (*e*.*g*., an axon, an axon collateral, and/or a dendrite), formation and extension of a spine, suppression of degeneration of a neuron, and suppression of cell death for a neuron. In this connection, pomotion of the extension of an axon collateral or a dendrite includes, for example, increase in the neurite length and promotion of branching.

A "neuron (neuronal cell)" is not particularly limited herein as long as the cell is a cell of neural lineage. The nervous system is classified into the central nervous system (the brain or the spinal cord) and the peripheral nervous system, and a neuron may be of the central or peripheral nervous system, unless otherwise specified. In this connection, a cell of neural lineage includes a cell which does not generate action potentials but connects and inputs to the nervous system (*e*.*g*., a photoreceptor cell). The stage of cell differentiation is not limited as long as the cell has an ability of differentiating into a neuron, and the maturation stage of the differentiated neuron is also not limited. For example, the cell may be a neural stem cell, a neural progenitor cell, an immature neuron, or a mature neuron. Also, the cell type of a neuron is not limited, and may be, for example, a dopaminergic neuron, a noradrenergic neuron, a serotonergic neuron, a glutamatergic neuron, a GABAergic neuron, a cholinergic neuron, or a histaminergic neuron. Unless otherwise specified, a neuron may be derived from a living body (*e*.*g*., brain), or may be an artificially produced neuron. Examples of a neuron derived from a living body include a neuron which exists or functions in any region (*e*.*g*., brain) of a living body and a neuron which is isolated from any region (*e*.*g*., brain) in a living body. Examples of an artificially produced neuron include a neuron which is derived from a (pluripotent) stem cell such as an ES cell or an iPS cell through induction of differentiation. In the present aspect, a neuron may be present *in vivo* (*e.g.,* intrinsically present in the brain or transplanted into the brain) or may be cultured *in vitro,* unless otherwise specified.

The term "a brain neuron" used herein refers to a neuron which can exist or function in any region of the brain, in the neuron described above. A region in which a brain neuron can exist or function in the brain is not limited. For example, such region may be cerebrum (*e*.*g*., cerebral cortex, cerebral white matter, or basal ganglia), cerebellum (*e*.*g*., cerebellar cortex or cerebellar nucleus), and/or brain stem (*e*.*g*., diencephalon, midbrain, pons, or medulla oblongata). Examples of a brain neuron include a neuron in the cerebral cortex, a neuron in the cerebellar cortex, and a neuron in the nucleus. Examples of said nucleus include a nucleus encompassing a plurality of nuclei, such as the basal ganglia. A suitable neuronherein is a brain neuron, and more preferably, a neuron in the cerebral cortex and/or a neuron in the midbrain. A neuron in the cerebral cortex is preferably a neuron in the primary motor cortex, and particularly preferably, an upper motor neuron in Layer V of the primary motor cortex. As a neuron in the midbrain, a neuron in the ventrotegmental area is preferable, and a dopaminergic neuron existing in the substantia nigra is particularly preferred.

The term "a plurality of" used herein refers to an integer equal to or greater than 2, such as 2 to 10, 2 to 7, 2 to 5, 2 to 4, or 2 to 3.

The term "a mutant protein" used herein refers to any protein having an amino acid sequence derived from the amino acid sequence of a wild-type protein and differing from the amino acid sequence of the wild-type protein. Examples of a mutant protein include a splicing variant, an SNP-based mutant, and an active fragment of a protein. Examples of a mutant protein can also include an artificial protein comprising an amino acid sequence differing from the amino acid sequence of the wild-type protein, which was prepared based on the amino acid sequence of the wild-type protein.

The term "active fragment" used herein refers to a polypeptide fragment comprising a partial region of a protein that retains an activity of 50% or more, 60% or more, 70% or more, 80% or more, 90% or more, 95% or more, 98% or more, or equivalent to or more than the activity of the full-length protein. The amino acid length of the active fragment is not particularly limited, as long as the fragment retains an activity of the protein.

The term "amino acid identity" used herein refers to the proportion (%) of the number of amino acid residues identical between the amino acid sequences of two polypeptides to be compared, relative to the total number of amino acid residues, when aligned as necessary by inserting a gap into one or both of the amino acid sequences as appropriate, so as to maximize the number of identical amino acid residues.

The term "(amino acid) substitution" used herein refers to a substitution within a group of conservative amino acids having similar properties in terms of charge, side chain, polarity, aromacity, and the like among 20 types of amino acid constituting a naturally-occurring protein. Examples of a substitution include a substitution within a group of uncharged polar amino acids having a low-polarity side chain (Gly, Asn, Gln, Ser, Thr, Cys, and Tyr), a group of branched amino acids (Leu, Val, and Ile), a group of neutral amino acids (Gly, Ile, Val, Leu, Ala, Met, and Pro), a group of neutral amino acids having a hydrophilic side chain (Asn, Gln, Thr, Ser, Tyr, and Cys), a group of acidic amino acids (Asp and Glu), a group of basic amino acids (Arg, Lys, and His), and a group of aromatic amino acids (Phe, Tyr, and Trp). An amino acid substitution within these groups is preferable because it is known that it is unlikely to cause changes in polypeptide properties.

### 1-3. Configuration

### 1-3-1. Component

An agent for activating a neuron according to the present invention comprises, as an essential active ingredient, a protein selected from the group consisting of each protein of LYZ, CD52, MPEG1, CCL2, CST7, RAC2, SERPINA3, ANOS1, KLK6, A2M, GM14295, SPP1, LGALS3BP, APOC1, PGrn, Ctsd, Ctss, Apod, and Sparc shown in the following Table 1 and Table 1', each protein shown in Tables 3, 5, 7, and 9, an active fragment of any of them, and an ortholog of any of them (which is referred to as a "a neuron-activating factor" herein; a neuron-activating factor may also be referred to as "a transplantation-assisting factor" as described in the second aspect below), or a nucleic acid encoding said protein, said active fragment, or said ortholog (which is referred to as "a nucleic acid encoding a neuron-activating factor" herein). For example, an agent for activating a neuron according to the present invention comprises, as an essential active ingredient, a protein selected from the group consisting of each protein of LYZ, CST7, CCL12, CD52, MPEG1, RAC2, SPP1, PGrn, Ctsd, Ctss, Apod, and Sparc, an active fragment of any thereof, and an ortholog of any thereof or a nucleic acid encoding said protein, said active fragment, or said ortholog. In an embodiment, an agent for activating neuron according to the present invention comprises, as an essential active ingredient, a protein selected from the group consisting of each protein of LYZ, SPP1, PGrn, Ctsd, Ctss, Apod, and Sparc described above, an active fragment of any thereof, an ortholog of any thereof, and a nucleic acid encoding said protein, said active fragment, or said ortholog. In a preferable embodiment, an agent for activating a neuron according to the present invention comprises, as an essential active ingredient, a protein selected from the group consisting of each protein of LYZ, SPP1, PGrn, and Sparc described above, an active fragment of any thereof, and an ortholog of any thereof or a nucleic acid encoding said protein, said active fragment, or said ortholog. In a more preferable embodiment, an agent for activating a neuron according to the present invention comprises, as an essential active ingredient, a protein selected from the group consisting of each protein of LYZ, SPP1, and PGrn described above, an active fragment of any thereof, and an ortholog of any thereof or a nucleic acid encoding said protein, said active fragment, or said ortholog. In a furthermore preferable embodiment, an agent for activating a neuron according to the present invention comprises, as an essential active ingredient, a protein selected from the group consisting of any protein of LYZ or PGrn described above, an active fragment thereof, and an ortholog thereof or a nucleic acid encoding said protein, said active fragment, or said ortholog. In a particularly preferable embodiment, an agent for activating a neuron according to the present invention comprises, as an essential active ingredient, a protein selected from the group consisting of LYZ described above, an active fragment thereof, and an ortholog thereof or a nucleic acid encoding said LYZ, said active fragment, or said ortholog. Individual active ingredients are specifically described below.

### (LYZ and LYZ ortholog)

Lysozyme (LYZ) may be a human wild-type LYZ protein consisting of the amino acid sequence as shown in SEQ ID NO: 2; a mutant LYZ protein having an amino acid sequence derived from the amino acid sequence as shown in SEQ ID NO: 2 in which one or a plurality of amino acids are deleted, substituted, or added, and having an activity equivalent to or higher than that of a human wild-type LYZ protein; or a mutant LYZ protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 2 and having an activity equivalent to or higher than that of a human wild-type LYZ protein.

An LYZ ortholog may be an ortholog in species other than human having 60% or higher, 70% or higher, 80% or higher, 90% or higher, 95% or higher, or 99% or higher, and lower than 100% identity to the amino acid sequence as shown in SEQ ID NO: 2 (e.g., a mouse LYZ2 protein consisting of the amino acid sequence as shown in SEQ ID NO: 1).

### (CD52 and CD52 ortholog)

CD52 may be a human wild-type CD52 protein consisting of the amino acid sequence as shown in SEQ ID NO: 4, a mutant CD52 protein having an amino acid sequence derived from the amino acid sequence as shown in SEQ ID NO: 4 in which one or a plurality of amino acids are deleted, substituted, or added, and having an activity equivalent to or higher than that of a human wild-type CD52 protein; or a mutant CD52 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 4 and having an activity equivalent to or higher than that of a human wild-type CD52 protein.

A CD52 ortholog may be an ortholog in species other than human comprising an amino acid sequence having 60% or higher, 70% or higher, 80% or higher, 90% or higher, 95% or higher, or 99% or higher, and lower than 100% identity to the amino acid sequence as shown in SEQ ID NO: 4 (*e*.*g*., a mouse CD52 protein consisting of the amino acid sequence as shown in SEQ ID NO: 3).

### (MPEG1 and MPEG1 ortholog)

Macrophage expressed gene 1 (MPEG1) may be a human wild-type MPEG1 protein consisting of the amino acid sequence as shown in SEQ ID NO: 7; a mutant MPEG1 protein having an amino acid sequence derived from the amino acid sequence as shown in SEQ ID NO: 7 in which one or a plurality of amino acids are deleted, substituted, or added, and having an activity equivalent to or higher than that of a human wild-type MPEG1 protein; or a mutant MPEG1 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 7 and having an activity equivalent to or higher than that of a human wild-type MPEG1 protein.

An MPEG1 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 7 (*e*.*g*., a mouse MPEG1 protein consisting of the amino acid sequence as shown in SEQ ID NO: 5 or 6).

### (CCL2 and CCL2 ortholog)

C-C motif chemokine ligand 2 (CCL2) may be a human wild-type CCL2 protein consisting of the amino acid sequence as shown in SEQ ID NO: 9; a mutant CCL2 protein having an amino acid sequence derived from the amino acid sequence as shown in SEQ ID NO: 9 in which one or a plurality of amino acids are deleted, substituted, or added, and having an activity equivalent to or higher than that of a human wild-type CCL2 protein; or a mutant CCL2 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 9 and having an activity equivalent to or higher than that of a human wild-type CCL2 protein.

A CCL2 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 9 (e.g., a mouse CCL12 protein consisting of the amino acid sequence as shown in SEQ ID NO: 8).

### (CST7 and CST7 ortholog)

Cystatin F (CST7) may be a human wild-type CST7 protein consisting of the amino acid sequence as shown in SEQ ID NO: 11; a mutant CST7 protein having an amino acid sequence derived from the amino acid sequence as shown in SEQ ID NO: 11 in which one or a plurality of amino acids are deleted, substituted, or added, and having an activity equivalent to or higher than that of a human wild-type CST7 protein; or a mutant CST7 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 11 and having an activity equivalent to or higher than that of a human wild-type CST7 protein.

A CST7 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 11 (*e*.*g*., a mouse CST7 protein consisting of the amino acid sequence as shown in SEQ ID NO: 10).

### (RAC2 and RAC2 ortholog)

The Ras family small GTPase 2 (RAC2) may be a human wild-type RAC2 protein consisting of the amino acid sequence as shown in SEQ ID NO: 13; a mutant RAC2 protein having an amino acid sequence derived from the amino acid sequence as shown in SEQ ID NO: 13 in which one or a plurality of amino acids are deleted, substituted, or added, and having an activity equivalent to or higher than that of a human wild-type RAC2 protein; or a mutant RAC2 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 13 and having an activity equivalent to or higher than that of a human wild-type RAC2 protein.

A RAC2 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 13 (*e*.*g*., a mouse RAC2 protein consisting of the amino acid sequence as shown in SEQ ID NO: 12).

### (SERPINA3 and SERPINA3 ortholog)

Serpin family A member 3 (SERPINA3) may be a human wild-type SERPINA3 protein consisting of the amino acid sequence as shown in SEQ ID NO: 15; a mutant SERPINA3 protein having an amino acid sequence derived from the amino acid sequence as shown in SEQ ID NO: 15 in which one or a plurality of amino acids are deleted, substituted, or added, and having an activity equivalent to or higher than that of a human wild-type SERPINA3 protein; or a mutant SERPINA3 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 15 and having an activity equivalent to or higher than that of a human wild-type SERPINA3 protein.

A SERPINA3 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 15 (*e*.*g*., a mouse SERPINA3N protein consisting of the amino acid sequence as shown in SEQ ID NO: 14).

### (ANOS1 and ANOS1 ortholog)

Anosmin 1 (ANOS1) may be a human wild-type ANOS1 protein consisting of the amino acid sequence as shown in SEQ ID NO: 17; a mutant ANOS1 protein having an amino acid sequence derived from the amino acid sequence as shown in SEQ ID NO: 17 in which one or a plurality of amino acids are deleted, substituted, or added, and having an activity equivalent to or higher than that of a human wild-type ANOS1 protein; or a mutant ANOS1 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 17 and having an activity equivalent to or higher than that of a human wild-type ANOS1 protein.

An ANOS1 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 17 (*e*.*g*., a mouse GM11428 protein consisting of the amino acid sequence as shown in SEQ ID NO: 16).

### (KLK6 and KLK6 ortholog)

Kallikrein related-peptidase 6 (KLK6) may be a human wild-type KLK6 protein consisting of the amino acid sequence as shown in any of SEQ ID NOs: 19 to 23; a mutant KLK6 protein having an amino acid sequence derived from the amino acid sequence as shown in any of SEQ ID NOs: 19 to 23 in which one or a plurality of amino acids are deleted, substituted, or added, and having an activity equivalent to or higher than that of a human wild-type KLK6 protein; or a mutant KLK6 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in any of SEQ ID NOs: 19 to 23 and having an activity equivalent to or higher than that of a human wild-type KLK6 protein.

A KLK6 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in any of SEQ ID NOs: 19 to 23 (*e*.*g*., a mouse KLK6 protein consisting of the amino acid sequence as shown in SEQ ID NO: 18).

### (A2M and A2M ortholog)

Alpha-2-macroglobulin (A2M) may be a human wild-type A2M protein consisting of the amino acid sequence as shown in any of SEQ ID NOs: 25 to 28; a mutant A2M protein having an amino acid sequence derived from the amino acid sequence as shown in any of SEQ ID NOs: 25 to 28 in which one or a plurality of amino acids are deleted, substituted, or added, and having an activity equivalent to or higher than that of a human wild-type A2M protein; or a mutant A2M protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in any of SEQ ID NOs: 25 to 28 and having an activity equivalent to or higher than that of a human wild-type A2M protein.

An A2M ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in any of SEQ ID NOs: 25 to 28 (*e*.*g*., a mouse A2M protein consisting of the amino acid sequence as shown in SEQ ID NO: 24).

### (GM14295 and GM14295 ortholog)

GM14295 (Predicted gene 1429) may be a mouse wild-type GM14295 protein consisting of the amino acid sequence as shown in SEQ ID NO: 29; a mutant GM14295 protein having an amino acid sequence derived from the amino acid sequence as shown in SEQ ID NO: 29 in which one or a plurality of amino acids are deleted, substituted, or added, and having an activity equivalent to or higher than that of a mouse wild-type GM14295 protein; or a mutant GM14295 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 29 and having an activity equivalent to or higher than that of a mouse wild-type GM14295 protein.

A GM14295 ortholog may be an ortholog in species other than mouse having an amino acid sequence having 60% or higher, 70% or higher, 80% or higher, 90% or higher, 95% or higher, or 99% or higher, and lower than 100% identity to the amino acid sequence as shown in SEQ ID NO: 29. No GM14295 ortholog is known in human.

### (SPP1 and SPP1 ortholog)

Secreted phosphoprotein 1 (SPP1; also referred to as "Osteopontin") may be a human wild-type SPP1 protein consisting of the amino acid sequence as shown in SEQ ID NO: 35 or 36; a mutant SPP1 protein having an amino acid sequence derived from the amino acid sequence as shown in SEQ ID NO: 35 or 36 in which one or a plurality of amino acids are deleted, substituted, or added, and having an activity equivalent to or higher than that of a human wild-type SPP1 protein; or a mutant SPP1 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 35 or 36 and having an activity equivalent to or higher than that of a human wild-type SPP1 protein.

An SPP1 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 35 or 36 (*e*.*g*., a mouse SPP1 protein consisting of the amino acid sequence as shown in any of SEQ ID NOs: 30 to 34).

### (LGALS3BP and LGALS3BP ortholog)

Galectin 3-binding protein (LGALS3BP) may be a human wild-type LGALS3BP protein consisting of the amino acid sequence as shown in SEQ ID NO: 38; a mutant LGALS3BP protein having an amino acid sequence derived from the amino acid sequence as shown in SEQ ID NO: 38 in which one or a plurality of amino acids are deleted, substituted, or added, and having an activity equivalent to or higher than that of the human wild-type LGALS3BP protein; or a mutant LGALS3BP protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 38 and having an activity equivalent to or higher than that of a human wild-type LGALS3BP protein.

An LGALS3BP ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 38 (*e*.*g*., a mouse LGALS3BP protein consisting of the amino acid sequence as shown in SEQ ID NO: 37).

### (APOC1 and APOC1 ortholog)

Apolipoprotein C1 (APOC1) may be a human wild-type APOC1 protein consisting of the amino acid sequence as shown in any of SEQ ID NOs: 41 to 44; a mutant APOC1 protein having an amino acid sequence derived from the amino acid sequence as shown in any of SEQ ID NOs: 41 to 44 in which one or a plurality of amino acids are deleted, substituted, or added, and having an activity equivalent to or higher than that of a human wild-type APOC1 protein; or a mutant APOC1 protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in any of SEQ ID NOs: 41 to 44 and having an activity equivalent to or higher than that of a human wild-type APOC1 protein.

An APOC1 ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in any of SEQ ID NOs: 41 to 44 (*e*.*g*., a mouse APOC1 protein consisting of the amino acid sequence as shown in SEQ ID NO: 39 or 40).

### (PGrn and PGrn ortholog)

Progranulin (PGrn or Grn) may be a human wild-type PGrn protein consisting of the amino acid sequence as shown in SEQ ID NO: 90; a mutant PGrn protein having an amino acid sequence derived from the amino acid sequence as shown in SEQ ID NO: 90 in which one or a plurality of amino acids are deleted, substituted, or added, and having an activity equivalent to or higher than that of a human wild-type PGrn protein; or a mutant PGrn protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 90 and having an activity equivalent to or higher than that of a human wild-type PGrn protein.

A PGrn ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 90 (*e*.*g*., a mouse PGrn protein consisting of the amino acid sequence as shown in SEQ ID NO: 89).

### (Ctsd and Ctsd ortholog)

The Cathepsin D (Ctsd) may be a human wild-type Ctsd protein consisting of the amino acid sequence as shown in SEQ ID NO: 92; a mutant Ctsd protein having an amino acid sequence derived from the amino acid sequence as shown in SEQ ID NO: 92 in which one or a plurality of amino acids are deleted, substituted, or added, and having an activity equivalent to or higher than that of a human wild-type Ctsd protein; or a mutant Ctsd protein having an amino acid sequence exhibiting 80% or higher, 85% or higher, preferably 90% or higher or 95% or higher, and more preferably 96% or higher, 97% or higher, 98% or higher, or 99% or higher identity to the amino acid sequence as shown in SEQ ID NO: 92 and having an activity equivalent to or higher than that of a human wild-type Ctsd protein.

A Ctsd ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 92 (*e*.*g*., a mouse Ctsd protein consisting of the amino acid sequence as shown in SEQ ID NO: 91).

### (Ctss and Ctss ortholog)

Cathepsin S (Ctss) may be a human wild-type Ctss protein consisting of the amino acid sequence as shown in SEQ ID NO: 95 or 96; a mutant Ctss protein having an amino acid sequence derived from the amino acid sequence as shown in SEQ ID NO: 95 or 96 in which one or a plurality of amino acids are deleted, substituted, or added, and having an activity equivalent to or higher than that of a human wild-type Ctss protein; or a mutant Ctss protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 95 or 96 and having an activity equivalent to or higher than that of a human wild-type Ctss protein.

A Ctss ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 95 or 96 (*e*.*g*., a mouse Ctss protein consisting of the amino acid sequence as shown in SEQ ID NO: 93 or 94).

### (Apod and Apod ortholog)

Apolipoprotein D (Apod) may be a human wild-type Apod protein consisting of the amino acid sequence as shown in SEQ ID NO: 100; a mutant Apod protein having an amino acid sequence derived from the amino acid sequence as shown in SEQ ID NO: 100 in which one or a plurality of amino acids are deleted, substituted, or added, and having an activity equivalent to or higher than that of a human wild-type Apod protein; or a mutant Apod protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in SEQ ID NO: 100 and having an activity equivalent to or higher than that of a human wild-type Apod protein.

An Apod ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in SEQ ID NO: 100 (*e*.*g*., a mouse Apod protein consisting of the amino acid sequence as shown in any of SEQ ID NOs: 97 to 99).

### (Sparc and Sparc ortholog)

The Sparc protein may be a human wild-type Sparc protein consisting of the amino acid sequence as shown in any of SEQ ID NOs: 103 to 105; a mutant Sparc protein having an amino acid sequence derived from the amino acid sequence as shown in any of SEQ ID NOs: 103 to 105 in which one or a plurality of amino acids are deleted, substituted, or added, and having an activity equivalent to or higher than that of a human wild-type Sparc protein; or a mutant Sparc protein having an amino acid sequence having 80% or higher, 85% or higher, preferably 90% or higher or 95% or higher, and more preferably 96% or higher, 97% or higher, 98% or higher, or 99% or higher identity to the amino acid sequence as shown in any of SEQ ID NOs: 103 to 105 and having an activity equivalent to or higher than that of a human wild-type Sparc protein.

An Sparc ortholog may be an ortholog in species other than human having an amino acid sequence having 60% or higher, 70% or higher, 80% or higher, 90% or higher, 95% or higher, or 99% or higher, and lower than 100% identity to the amino acid sequence as shown in any of SEQ ID NOs: 103 to 105 (*e*.*g*., a mouse Sparc protein consisting of the amino acid sequence as shown in SEQ ID NO: 101 or 102).

Neuron-activating factors, NCBI accession numbers corresponding thereto, and SEQ ID NOs thereof are exemplified in Table 1 and Table 1' below. As described in the second aspect below, neuron-activating factors exemplified in Table 1 and Table 1' can be referred to as "transplantation-assisting factors."

### [Table 1]

**Table 1: Examples of neuron-activating factors**

| Human | | | Mouse | | |
|---|---|---|---|---|---|
| Name | NCBI Accession No. | SEQ ID NO: | Name | NCBI Accession No. | SEQ ID NO: |
| LYZ | NP_000230.1 | 2 | LYZ2 | NP_059068.1 | 1 |
| CD52 | NP_001794.2 | 4 | CD52 | NP_038734.1 | 3 |
| MPEG1 | NP_001034485.1 | 7 | MPEG1 | NP_001361597.1 | 5 |
| | | | | NP_034951.2 | 6 |
| CCL2 | NP_002973.1 | 9 | CCL12 | NP_035461.2 | 8 |
| CST7 | NP_003641.3 | 11 | CST7 | NP_034107.2 | 10 |
| RAC2 | NP_002863.1 | 13 | RAC2 | NP_033034.1 | 12 |
| SERPINA3 | NP_001076.2 | 15 | SERPINA3N | NP_033278.2 | 14 |
| ANOS1 | NP_000207.2 | 17 | GM11428 | NP_001075426.1 | 16 |
| KLK6 | NP_001012982.1 | 19 | KLK6 | NP_001158169.1 | 18 |
| | NP_001012983.1 | 20 | | | |
| | NP_001306877.1 | 21 | | | |
| | NP_001306878.1 | 22 | | | |
| | NP_002765.1 | 23 | | | |
| A2M | NP_000005.3 | 25 | A2M | NP_783327.2 | 24 |
| | NP_001334352.2 | 26 | | | |
| | NP_001334353.2 | 27 | | | |
| | NP_001334354.2 | 28 | | | |
| Unknown | - | - | GM14295 | NP_001191986.1 | 29 |
| SPP1 | NP_000573.1 | 35 | SPP1 | NP_001191130.1 | 30 |
| | NP_001035147.1 | 36 | | NP_001191131.1 | 31 |
| | | | | NP_001191132.1 | 32 |
| | | | | NP_001191162.1 | 33 |
| | | | | NP_033289.2 | 34 |
| LGALS3BP | NP_005558.1 | 38 | LGALS3BP | NP_035280.1 | 37 |
| APOC1 | NP_001307994.1 | 41 | APOC 1 | NP_001103479.1 | 39 |
| | NP_001307995.1 | 42 | | NP_031495.1 | 40 |
| | NP_001366616.1 | 43 | | | |
| | NP_001636.1 | 44 | | | |

**Table 1': Examples of neuron-activating factors**

| Human | | | Mouse | | |
|---|---|---|---|---|---|
| Name | NCBI Accession No. | SEQ ID NO: | Name | NCBI Accession No. | SEQ ID NO: |
| PGrn(Grn) | NP_002078.1 | 90 | PGrn(Grn) | NP_032201.3 | 89 |
| Ctsd | NP_001900.1 | 92 | Ctsd | NP_034113.1 | 91 |
| Ctss | NP_004070.3 | 95 | Ctss | NP_001254624.2 | 93 |
| | NP_001186668.1 | 96 | | NP_067256.4 | 94 |
| Apod | NP_001638.1 | 100 | | NP_001288282.1 | 97 |
| | | | Apod | NP_001288283.1 | 98 |
| | | | | NP_031496.2 | 99 |
| Sparc | NP_001296372.1 | 103 | | NP_001277746.1 | 101 |
| | NP_001296373.1 | 104 | Sparc | NP_033268.1 | 102 |
| | NP_003109.1 | 105 | | | |

The neuron-activating factors described above may be introduced into a cell in accordance with a technique, such as lipofection, fusion with a cell-penetrating peptide (e.g., HIV-derived TAT, polyarginine, BP100, Penetratin, pVEC, pAntp, VP22, Transportan, R7, MPG, and Pep-1), or microinjection.

### (A nucleic acid encoding said protein, said active fragment, or said ortholog)

The term "a nucleic acid encoding said protein, said active fragment, or said ortholog" (which is also referred herein to as "a nucleic acid encoding a neuron-activating factor") refers to a nucleic acid encoding any of the neuron-activating factors described above. A nucleic acid encoding a neuron-activating factor is a nucleic acid which encodes LYZ, CD52, MPEG1, CCL2, CST7, RAC2, SERPINA3, ANOS1, KLK6, A2M, GM14295, SPP1, LGALS3BP, APOC1, PGrn, Ctsd, Ctss, Apod, or Sparc, a mutant protein described above which is derived from any of them, an active fragment of any of them, or an ortholog described above for any of them, regardless of whether it is DNA or RNA. DNA encoding a neuron-activating factor may be a gene encoding the neuron-activating factor or cDNA prepared using a trascriptional product thereof as a template. RNA encoding a neuron-activating factor encompasses both mRNA precursor (pre-mRNA) and mature mRNA, but substantially means mature mRNA.

Specific examples of DNA encoding a neuron-activating factor (cDNA encoding a neuron-activating factor) include DNA encoding a human wild-type LYZ protein which consists of the base sequence as shown in SEQ ID NO: 46; DNA encoding a mouse wild-type LYZ2 protein which consists of the base sequence as shown in SEQ ID NO: 45; DNA encoding a human wild-type CD52 protein which consists of the base sequence as shown in SEQ ID NO: 48; DNA encoding a mouse wild-type CD52 protein which consists of the base sequence as shown in SEQ ID NO: 47; DNA encoding a human wild-type MPEG1 protein which consists of the base sequence as shown in SEQ ID NO: 51; DNA encoding a mouse wild-type MPEG1 protein which consists of the base sequence as shown in SEQ ID NO: 49 or 50; DNA encoding a human wild-type CCL2 protein which consists of the base sequence as shown in SEQ ID NO: 53; DNA encoding a mouse wild-type CCL12 protein which consists of the base sequence as shown in SEQ ID NO: 52; DNA encoding a human wild-type CST7 protein which consists of the base sequence as shown in SEQ ID NO: 55; DNA encoding a mouse wild-type CST7 protein which consists of the base sequence as shown in SEQ ID NO: 54; DNA encoding a human wild-type RAC2 protein which consists of the base sequence as shown in SEQ ID NO: 57; DNA encoding a mouse wild-type RAC2 protein which consists of the base sequence as shown in SEQ ID NO: 56; DNA encoding a human wild-type SERPINA3 protein which consists of the base sequence as shown in SEQ ID NO: 59; DNA encoding a mouse wild-type SERPINA3N protein which consists of the base sequence as shown in SEQ ID NO: 58; DNA encoding a human wild-type ANOS1 protein which consists of the base sequence as shown in SEQ ID NO: 61; DNA encoding a mouse wild-type GM11428 protein which consists of the base sequence as shown in SEQ ID NO: 60; DNA encoding a human wild-type KLK6 protein which consists of the base sequence as shown in any of SEQ ID NOs: 63 to 67; DNA encoding a mouse wild-type KLK6 protein which consists of the base sequence as shown in SEQ ID NO: 62; DNA encoding a human wild-type A2M protein which consists of the base sequence as shown in any of SEQ ID NOs: 69 to 72; DNA encoding a mouse wild-type A2M protein which consists of the base sequence as shown in SEQ ID NO: 68; DNA encoding a mouse wild-type GM14295 protein which consists of the base sequence as shown in SEQ ID NO: 73; DNA encoding a human wild-type SPP1 protein which consists of the base sequence as shown in SEQ ID NO: 79 or 80; DNA encoding a mouse wild-type SPP1 protein which consists of the base sequence as shown in any of SEQ ID NOs: 74 to 78; DNA encoding a human wild-type LGALS3BP protein which consists of the base sequence as shown in SEQ ID NO: 82; DNA encoding a mouse wild-type LGALS3BP protein which consists of the base sequence as shown in SEQ ID NO: 81; DNA encoding a human wild-type APOC1 protein which consists of the base sequence as shown in any of SEQ ID NOs: 85 to 88; DNA encoding a mouse wild-type APOC1 protein which consists of the base sequence as shown in SEQ ID NO: 83 or 84; DNA encoding a human wild-type PGrn protein which consists of the base sequence as shown in SEQ ID NO: 107; DNA encoding a mouse wild-type PGrn protein which consists of the base sequence as shown in SEQ ID NO: 106; DNA encoding a human wild-type Ctsd protein which consists of the base sequence as shown in SEQ ID NO: 109; DNA encoding a mouse wild-type Ctsd protein which consists of the base sequence as shown in SEQ ID NO: 108; DNA encoding a human wild-type Ctss protein which consists of the base sequence as shown in SEQ ID NO: 112 or 113; DNA encoding a mouse wild-type Ctss protein which consists of the base sequence as shown in SEQ ID NO: 110 or 111; DNA encoding a human wild-type Apod protein which consists of the base sequence as shown in SEQ ID NO: 117; DNA encoding a mouse wild-type Apod protein which consists of the base sequence as shown in any of SEQ ID NOs: 114 to 116; DNA encoding a human wild-type Sparc protein which consists of the base sequence as shown in any of SEQ ID NOs: 120 to 122; and DNA encoding a mouse wild-type Sparc protein which consists of the base sequence as shown in SEQ ID NO: 118 or 119.

An example of mRNA encoding a neuron-activating factor is mRNA comprising, as a coding region (translated region), a base sequence derived from any of the base sequences described above in which t (thymine) is substituted with u (uracil). mRNA encoding a neuron-activating factor may comprise, in addition to said coding region, a 5'-terminal cap structure, a 3'-terminal poly A chain, a 5' untranslated region (5' UTR) upstream of the start codon, and/or a 3' untranslated region (3' UTR) downstream of the stop codon. 5' UTR and/or 3' UTR may comprise a sequence for adjusting the amount of translation from mRNA. For example, 3' UTR may comprise a sequence to increase the volume of translation from mRNA (e.g., the woodchuck hepatitis virus post-transcriptional regulatory element (WPRE)).

Nucleic acids encoding a neuron-activating factor, NCBI accession numbers corresponding thereto, and SEQ ID NOs are exemplified in Table 2 and Table 2' below. As described in the second aspect below, a nucleic acid encoding neuron-activating factor exemplified in Table 2 and Table 2' can be referred to as "a nucleic acid encoding a transplantation-assisting factor."

### [Table 2]

**Table 2: Examples of nucleic acids encoding a neuron-activating factor**

| Human | | | Mouse | | |
|---|---|---|---|---|---|
| Name | NCBI Accession No. | SEQ ID NOs | Name | NCBI Accession No. | SEQ ID NOs |
| LYZ | NM_000239.3 | 46 | LYZ2 | NM_017372.3 | 45 |
| CD52 | NM_001803.3 | 48 | CD52 | NM_013706.2 | 47 |
| MPEG1 | NM_001039396.2 | 51 | MPEG1 | NM_001374668.1 | 49 |
| | | | | NM_010821.2 | 50 |
| CCL2 | NM_002982.4 | 53 | CCL12 | NM_011331.3 | 52 |
| CST7 | NM_003650.4 | 55 | CST7 | NM_009977.3 | 54 |
| RAC2 | NM_002872.5 | 57 | RAC2 | NM_009008.3 | 56 |
| SERPINA3 | NM_001085.5 | 59 | SERPINA3N | NM_009252.2 | 58 |
| ANOS1 | NM_000216.4 | 61 | GM11428 | NM_001081957.1 | 60 |
| KLK6 | NM_001012964.3 | 63 | KLK6 | NM_001164697.1 | 62 |
| | NM_001012965.3 | 64 | | | |
| | NM_001319948.2 | 65 | | | |
| | NM_001319949.2 | 66 | | | |
| | NM_002774.4 | 67 | | | |
| A2M | NM_000014.6 | 69 | A2M | NM_175628.3 | 68 |
| | NM_001347423.2 | 70 | | | |
| | NM_001347424.2 | 71 | | | |
| | NM_001347425.2 | 72 | | | |
| Unknown | - | - | GM14295 | NM_001205057.2 | 73 |
| | NM_000582.3 | 79 | SPP1 | NM_001204201.1 | 74 |
| SPP1 | NM_001040058.2 | 80 | | NM_001204202.1 | 75 |
| | | | | NM_001204203.1 | 76 |
| | | | | NM_001204233.1 | 77 |
| | | | | NM_009263.3 | 78 |
| LGALS3BP | NM_005567.4 | 82 | LGALS3BP | NM_011150.3 | 81 |
| APOC1 | NM_001321065.2 | 85 | APOC1 | NM_001110009.2 | 83 |
| | NM_001321066.2 | 86 | | NM_007469.5 | 84 |
| | NM_001379687.1 | 87 | | | |
| | NM_001645.5 | 88 | | | |

**Table 2': Examples of nucleic acids encoding a neuron-activating factor**

| Human | | | Mouse | | |
|---|---|---|---|---|---|
| Name | NCBI Accession No. | SEQ ID NOs | Name | NCBI Accession No. | SEQ ID NOs |
| PGrn (Grn) | NM_002087.4 | 107 | Grn (Grn) | NM_008175.5 | 106 |
| Ctsd | NM_001909.5 | 109 | Ctsd | NM_009983.3 | 108 |
| Ctss | NM_004079.5 | 112 | Ctss | NM_001267695.2 | 110 |
| | NM_001199739.2 | 113 | | NM_021281.3 | 111 |
| Apod | NM_001647.4 | 117 | | NM_001301353.1 | 114 |
| | | | Apod | NM_001301354.1 | 115 |
| | | | | NM_007470.4 | 116 |
| Sparc | NM_001309443.2 | 120 | Sparc | NM_001290817.1 | 118 |
| | NM_001309444.2 | 121 | | NM_009242.5 | 119 |
| | NM_003118.4 | 122 | | | |

In a neuron-activating factor according to the present invention, a nucleic acid encoding a neuron-activating factor may be DNA or RNA. When a nucleic acid is in the form of DNA, it may be contained in a gene expression vector. When a nucleic acid is in the form of DNA, it may be introduced into a cell by means of, for example, electroporation, lipofection, liposome, or microinjection.

The term "gene expression vector" refers to an expression unit which contains a gene or gene fragment in a state which allows for expression thereof and is capable of controlling the expression of the gene or the like.

"A state which allows for expression" in the present aspect means that a gene or the like encoding a neuron-activating factor is positioned in a downstream region, which is under the control of a promoter. Accordingly, in a cell into which a vector is introduced (e.g., a neuron), expression of a neuron-activating factor is induced by a promoter.

In the present aspect, various gene expression vectors which can be replicated and allows for expression in the body of a subject (*e*.*g*., a neuron) can be used. Examples thereof include a viral vector, a plasmid vector, and an artificial chromosome vector. A viral vector is not particularly limited, provided that it can infect a cell (*e*.*g*., a neuron) and express a neuron-activating factor in the same cell. Examples thereof include adenovirus vector, adeno-associated virus (AAV) vector, retrovirus vector, lentivirus vector, and Sendai virus vector. A plasmid vector is not particularly limited, provided that it is capable of replication in a mammalian cell. Specific examples of plasmid vectors include pCMV6-XL3, pEGFP-C1, pGBT-9, pcDNA, pcDM8, and pREP4. Examples of artificial chromosome vectors include human artificial chromosome (HAC), yeast artificial chromosome (YAC) , and bacterial artificial chromosome (BAC or PAC).

A promoter is a promoter having an activity of inducing gene expression in a cell (*e.g.,* a neuron). Examples thereof include CMV promoter (CMV-IE promoter), LTR promoter, SV40 promoter, RSV promoter, HSV-TK promoter, EF1α promoter, Ub promoter, metallothionein promoter, SRα promoter, and CAG promoter.

A gene expression vector may comprise, for example, a regulatory sequence other than a promoter and/or a reporter gene. Examples of a regulatory sequence include an expression regulatory sequence (*e.g.,* an enhancer, a ribosome binding sequence, a terminator, and a poly A signal), an intron sequence, a nuclease recognition sequence (*e.g.,* a restriction enzyme recognition sequence, a loxP sequence recognized by Cre recombinase, a sequence targeted by an artificial nuclease, such as ZFN or TALEN, or a sequence targeted by the CRISPR/Cas9 system), and a replication origin sequence (*e.g.,* an SV40 replication origin sequence). Examples of a reporter gene include a gene encoding a fluorescent protein, such as GFP and RFP, and a luciferase gene.

In order to insert a nucleic acid encoding a gene into a chromosome or, if necessary, to remove a nucleic acid inserted into the chromosome, a gene expression vector may comprise a transposon sequence upstream and downstream of an expression cassette (a gene expression unit comprising a promoter, a gene sequence, and a terminator). A transposon sequence is not particularly limited, and an example thereof is piggyBac. In order to introduce an expression cassette into a chromosome using a transposon, it is desirable that a transposase is introduced into the same cell together with a vector comprising the expression cassette. In the present invention, a transposase may be introduced by incorporating a nucleic acid encoding the transposase into the above-mentioned vector, or incorporating the nucleic acid encoding the transposase into another vector and simultaneously introducing them into a cell. Alternatively, a gene product encoding the transposase may be introduced directly. In the present invention, a transposase is preferably a transposase corresponding to the transposon sequence described above, and more preferably a piggyBac transposase.

When a nucleic acid is in the form of RNA, it may be introduced into a cell by means of, for example, electroporation, lipofection, or microinjection.

In an embodiment, an agent for activating a neuron may comprise active ingredients of mutiple types or in multiple forms.

In another embodiment, a neuron-activating factor used in an agent for activating a neuron according to the present invention may be a protein selected from the group consisting of the proteins shown in the following Tables 3, 5, 7, and 9, an active fragment of any of them, and an ortholog of any of them. Additionally, nucleic acids encoding the proteins shown in Tables 3, 5, and 7 and NCBI accession numbers corresponding thereto are shown in the following Tables 4, 6, and 8. As described in the second aspect described below, a neuron-activating factor exemplified in Tables 3, 5, 7, and 9 can also be referred to as a "transplantation-assisting factor." The nucleic acid encoding a neuron-activating factor shown in Tables 4, 6, and 8 can also be referred to as a "nucleic acid encoding a transplantation-assisting factor."

### [Table 3]

**Table 3: Further examples of neuron-activating factors (1)**

| Human | | Mouse | |
|---|---|---|---|
| Name | NCBI Accession No. | Name | NCBI Accession No. |
| Csf2ra | NP_001155001.1 | Csf2ra | NP_034100.2 |
| | NP_001155002.1 | | XP_036017313.1 |
| | NP_001155003.1 | | XP_036017312.1 |
| | NP_001155004.1 | | XP_017173541.1 |
| | NP_001366082.1 | | XP_011245433.1 |
| | NP_001366083.1 | | XP_011245432.1 |
| | NP_001366084.1 | | XP_011245434.1 |
| | NP_001366085.1 | | XP_036017311.1 |
| | NP_001366087.1 | | |
| | NP_001366088.1 | | |
| | NP_001366089.1 | | |
| | NP_001366090.1 | | |
| | NP_001366091.1 | | |
| | NP_001366092.1 | | |
| | NP_001366093.1 | | |
| | NP_001366094.1 | | |
| | NP_001366095.1 | | |
| | NP_001366096.1 | | |
| | NP_001366097.1 | | |
| | NP_001366098.1 | | |
| | NP_006131.2 | | |
| | NP_758448.1 | | |
| | NP_758449.1 | | |
| | NP_758450.1 | | |
| | NP_758452.1 | | |
| Lag3 | NP_002277.4 | Lag3 | NP_032505.1 |
| Cxcl16 | NP_001094282.2 | Cxcl16 | NP_075647.3 |
| | NP_001373738.1 | | |
| Cd44 | NP_000601.3 | Cd44 | NP_001034239.1 |
| | NP_001001389.1 | | NP_001034240.1 |
| | NP_001001390.1 | | NP_001171256.1 |
| | NP_001001391.1 | | NP_001171257.1 |
| | NP_001001392.1 | | NP_001171258.1 |
| | NP_001189484.1 | | NP_033981.2 |
| | NP_001189485.1 | | |
| | NP_001189486.1 | | |
| Tnfrsf1a | NP_001056.1 | Tnfrsf1a | NP_035739.2 |
| | NP_001333020.1 | | |
| | NP_001333021.1 | | |
| Csfl | NP_000748.4 | Csfl | NP_001107001.1 |
| | NP_757349.2 | | NP_001107002.1 |
| | NP_757350.2 | | NP_031804.3 |
| | NP_757351.2 | | |
| Tnfrsflb | NP_001057.1 | Tnfrsf1b | NP_035740.2 |

### [Table 4]

**Table 4: Further examples of nucleic acids encoding neuron-activating factors (1)**

| Human | | Mouse | |
|---|---|---|---|
| Name | NCBI Accession No. | Name | NCBI Accession No. |
| | NM_001161529.2 | Csf2ra | NM_009970.2 |
| | NM_001161530.2 | | XM_036161420.1 |
| | NM_001161531.2 | | XM_036161419.1 |
| | NM_001161532.2 | | XM_017318052.3 |
| | NM-001379153.1 | | XM_011247131.4 |
| | NM_001379154.1 | | XM_011247130.4 |
| | NM_001379155.1 | | XM_011247132.4 |
| | NM_001379156.1 | | XM_036161418.1 |
| | NM_001379158.1 | | |
| | NM_001379159.1 | | |
| | NM_001379160.1 | | |
| | NM_001379161.1 | | |
| Csf2ra | NM_001379162.1 | | |
| | NM_001379163.1 | | |
| | NM_001379164.1 | | |
| | NM_001379165.1 | | |
| | NM_001379166.1 | | |
| | NM_001379167.1 | | |
| | NM_001379168.1 | | |
| | NM_001379169.1 | | |
| | NM_006140.6 | | |
| | NM_172245.4 | | |
| | NM_172246.4 | | |
| | NM_172247.3 | | |
| | NM_172249.4 | | |
| Lag3 | NM_002286.6 | Lag3 | NM_008479.2 |
| Cxcl16 | NM_001100812.2 | Cxcl16 | NM_023158.7 |
| | NM_001386809.1 | | |
| Cd44 | NM_000610.4 | Cd44 | NM_001039150.1 |
| | NM_001001389.2 | | NM_001039151.1 |
| | NM_001001390.2 | | NM_001177785.1 |
| | NM_001001391.2 | | NM_001177786.1 |
| | NM_001001392.2 | | NM_001177787.1 |
| | NM_001202555.2 | | NM_009851.2 |
| | NM_001202556.2 | | |
| | NM_001202557.2 | | |
| Tnfrsf1a | NM 001065.4 | Tnfrsf1a | NM 011609.4 |
| | NM_001346091.2 | | |
| | NM_001346092.2 | | |
| Csf1 | NM_000757.6 | Csfl | NM_001113529.1 |
| | NM_172210.3 | | NM_001113530.1 |
| | NM_172211.4 | | NM_007778.4 |
| | NM_172212.3 | | |
| Tnfrsflb | NM_001066.3 | Tnfrsf1b | NM_011610.3 |

### [Table 5]

**Table 5: Further examples of neuron-activating factors (2)**

| Human | | Mouse | |
|---|---|---|---|
| Name | NCBI Accession No. | Name | NCBI Accession No. |
| Apoc2 | NP_000474.2 | Apoc2 | NP_001264873.1 |
| | | | NP_001296724.1 |
| Cd5l | NP_001334627.1 | Cd5l | NP_033820.2 |
| | NP_005885.1 | | |
| Cxcl 10 | NP_001556.2 | Cxcl10 | NP_067249.1 |
| Lgals3 | NP_001344607.1 | Lgals3 | NP_001139425.1 |
| | NP_002297.2 | | NP_034835.1 |
| Cxcl5 | NP_002985.1 | Cxcl5 | NP_033167.2 |
| C4b | NP_001002029.3 | C4b | NP_033910.2 |
| Ly86 | NP_004262.1 | Ly86 | NP_034875.1 |
| Isg 15 | NP_005092.1 | Isg15 | NP_056598.2 |
| Col3a1 | NP_000081.2 | Col3a1 | NP_034060.2 |
| C1qa | NP_001334394.1 | C1qa | NP_031598.2 |
| | NP_001334395.1 | | |
| | NP_057075.1 | | |
| C1qb | NP_000482.3 | C1qb | NP_033907.1 |
| | NP_001358113.2 | | |
| | NP_001365085.1 | | |
| Ctsd | NP_001900.1 | Ctsd | NP_034113.1 |
| C1qc | NP_001107573.1 | C1qc | NP_031600.2 |
| | NP_001334548.1 | | |
| | NP_001334549.1 | | |
| | NP_758957.2 | | |
| Cpxm 1 | NP_001171628.1 | Cpxm1 | NP_062670.2 |
| | NP_062555.1 | | |
| Grn | NP_002078.1 | Grn | NP_032201.3 |
| Lgals9 | NP_001317092.1 | Lgals9 | NP_001152773.1 |
| | NP_002299.2 | | NP_034838.2 |
| | NP_033665.1 | | |
| Sparc | NP_001296372.1 | Sparc | NP_001277746.1 |
| | NP_001296373.1 | | NP_033268.1 |
| | | | |
| Apod | NP_001638.1 | Apod | NP_001288282.1 |
| | | | NP_001288283.1 |
| | | | NP_031496.2 |
| Olfml3 | NP_001273281.1 | Olfml3 | NP_598620.2 |
| | NP_001273282.1 | | |
| | NP_064575.1 | | |
| Serping1 | NP_000053.2 | Serping1 | NP_033906.2 |
| | NP_001027466.1 | | |
| Col18a1 | NP_000079.2 | Col18a1 | NP_031768.2 |
| Ttr | NP_000362.1 | Ttr | XP_030106272.1 |

### [Table 6]

**Table 6: Further examples of nucleic acids encoding neuron-activating factors (2)**

| Human | | Mouse | |
|---|---|---|---|
| Name | NCBI Accession No. | Name | NCBI Accession No. |
| Apoc2 | NM_000483.5 | Apoc2 | NM_001277944.1 |
| | | | NM_001309795.1 |
| Cd5l | NM_001347698.2 | Cd5l | NM_009690.2 |
| | NM_005894.3 | | |
| Cxcl10 | NM_001565.4 | Cxcl 10 | NM_021274.2 |
| Lgals3 | NM_001357678.2 | Lgals3 | NM_001145953.1 |
| | NM_002306.4 | | NM_010705.3 |
| Cxcl5 | NM_002994.5 | Cxcl5 | NM_009141.3 |
| C4b | NM_001002029.4 | C4b | NM_009780.2 |
| Ly86 | NM_004271.4 | Ly86 | NM_010745.2 |
| Isg 15 | NM_005101.4 | Isg 15 | NM_015783.3 |
| Col3a1 | NM_000090.4 | Col3a1 | NM_009930.2 |
| C1qa | NM_001347465.2 | C1qa | NM_007572.2 |
| | NM_001347466.2 | - | |
| | NM_015991.4 | | |
| C1qb | NM_000491.5 | C1qb | NM_009777.3 |
| | NM_001371184.3 | - | |
| | NM_001378156.1 | | |
| Ctsd | NM_001909.5 | Ctsd | NM_009983.3 |
| C1qc | NM_001114101.3 | C1qc | NM_007574.2 |
| | NM_001347619.2 | | |
| | NM_001347620.2 | | |
| | NM_172369.5 | | |
| Cpxm 1 | NM_001184699.2 | Cpxm1 | NM_019696.2 |
| | NM_019609.5 | | |
| Grn | NM_002087.4 | Grn | NM_08175.5 |
| Lgals9 | NM_001330163.2 | Lgals9 | NM_001159301.1 |
| | NM_002308.4 | | NM_010708.2 |
| | NM_009587.3 | | |
| Sparc | NM_001309443.2 | Sparc | NM_001290817.1 |
| | NM_001309444.2 | | NM_009242.5 |
| | NM_003118.4 | | |
| Apod | NM_001647.4 | Apod | NM_001301353.1 |
| | | | NM_001301354.1 |
| | | | NM_007470.4 |
| Olfml3 | NM_001286352.3 | Olfml3 | NM_133859.2 |
| | NM_001286353.3 | | |
| | NM_020190.5 | | |
| Serping 1 | NM_000062.3 | Serping1 | NM 009776.3 |
| | MM_001032295.2 | | |
| Col18a1 | NM_000088.4 | Col18a1 | NM_007742.4 |
| Ttr | NM_000371.4 | Ttr | XM_030250412.1 |

### [Table 7]

**Table 7: Further examples of neuron-activating factors (3)**

| Human | | Mouse | |
|---|---|---|---|
| Name | NCBI Accession No. | Name | NCBI Accession No. |
| Lilrb4 | NP_001265355.2 | Lilrb4 | NP_001278823.1 |
| | NP_001265356.2 | | NP 038560.1 |
| | NP_001265357.2 | | |
| | NP_001265358.2 | | |
| | NP_001265359.2 | | |
| Clec7a | NP_072092.2 | Clec7a | NP_001296566.1 |
| | NP_922938.1 | | NP_064392.2 |
| | NP_922939.1 | | |
| | NP_922940.1 | | |
| | NP_922941.1 | | |
| | NP_922945.1 | | |
| Tlr2 | NP_001305716.1 | Tlr2 | NP_036035.3 |
| | NP_001305718.1 | | |
| | NP_001305719.1 | | |
| | NP_001305720.1 | | |
| | NP_001305722.1 | | |
| | NP_001305724.1 | | |
| | NP_001305725.1 | | |
| | NP_003255.2 | | |
| Gpnmb | NP_001005340.1 | Gpnmb | NP_444340.3 |
| | NP_002501.1 | | |
| C3ar1 | NP_001313404.1 | C3ar1 | NP_033909.1 |
| | NP_001313406.1 | | |
| | NP_004045.1 | | |
| Ecel1 | NP_001277716.1 | Ecel1 | NP_001264854.1 |
| | NP_004817.2 | | NP_067281.2 |
| Tmem106a | NP_001278515.1 | Tmem106a | NP_001346254.1 |
| | NP_001278516.1 | | NP_001346255.1 |
| | NP_001278517.1 | | NP_001346256.1 |
| | NP_659478.1 | | NP_659079.1 |
| Ms4a7 | NP_067024.1 | Ms4a7 | NP_001020781.1 |
| | NP_996821.1 | | NP_001263327.1 |
| | NP_996822.1 | | NP_082112.3 |
| | NP_996823.1 | | |
| Cd48 | NP_001242959.1 | Cd48 | NP_001347696.1 |
| | NP_001769.2 | | NP_031675.1 |
| Ifi30 | NP_006323.2 | Ifi30 | NP_075552.2 |
| Cd180 | NP_005573.2 | Cd180 | NP_001347448.1 |
| | | | NP_032559.2 |
| Fcgr2b | NP_001002273.1 | Fcgr2b | NP_001070657.1 |
| | NP_001002274.1 | | NP_034317.1 |
| | NP_001002275.1 | | |
| | NP_001177757.1 | | |
| | NP_001372929.1 | | |
| | NP_001372930.1 | | |
| | NP_001372931.1 | | |
| | NP_001372932.1 | | |
| | NP_001372933.1 | | |
| | NP_001372934.1 | | |
| | NP_001372935.1 | | |
| | NP_003992.3 | | |
| Hvcn 1 | NP_001035196.1 | Hvcn1 | NP_001035954.1 |
| | NP_001243342.1 | | NP_001346383.1 |
| | NP_115745.2 | | NP 083028.1 |
| Bst2 | NP_004326.1 | Bst2 | NP_932763.1 |
| Rtp4 | NP_071430.2 | Rtp4 | XP 036015995.1 |
| | | | XP_030105116.1 |
| | | | XP_006522554.1 |
| | | | XP_030105117.1 |
| Trem2 | NP_001258750.1 | Trem2 | NP_001259007.1 |
| | NP_061838.1 | | NP_112544.1 |
| Cd68 | NP_001035148.1 | Cd68 | NP_001277987.1 |
| | NP_001242.2 | | |
| Hk2 | NP_000180.2 | Hk2 | NP_038848.1 |
| | NP_001358454.1 | | |
| Kcne5 | NP_036414.1 | Kcnell | NP_067462.1 |
| Abca1 | NP_005493.2 | Abca1 | NP_038482.3 |
| Icam 1 | NP_000192.2 | Icam1 | NP_034623.1 |
| P2ry6 | NP_001264133.1 | P2ry6 | NP_898991.1 |
| | NP_001264134.1 | | XP_036008888.1 |
| | NP_001264135.1 | | XP_011240041.1 |
| | NP_001264136.1 | | XP_006507703.1 |
| | NP_001264137.1 | | |
| | NP_789766.1 | | |
| | NP_789767.1 | | |
| | NP_789768.1 | | |
| Tbxas1 | NP_001052.3 | Tbxas1 | NP_035669.3 |
| | NP_001124438.2 | | |
| | NP_001159725.2 | | |
| | NP_001159726.1 | | |
| | NP_001300957.1 | | |
| | NP_001353466.1 | | |
| | NP_001353467.1 | | |
| | NP_112246.3 | | |
| Slc37a2 | NP_001138762.1 | Slc37a2 | NP_001139432.1 |
| | NP_938018.1 | | NP_064654.3 |
| Hmox1 | NP_002124.1 | Hmox1 | NP_034572.1 |
| Epsti1 | NP_001002264.1 | Epsti1 | NP_083771.1 |
| | NP_001317472.1 | | NP_849147.1 |
| | NP_001318157.1 | | |
| | NP_150280.1 | | |
| Slc15a3 | NP_057666.1 | Slc15a3 | NP 075531.2 |
| Cd74 | NP_001020329.1 | Cd74 | NP_001036070.1 |
| | NP_001020330.1 | | NP_034675.1 |
| | NP_001351012.1 | | |
| | NP_001351013.1 | | |
| | NP_004346.1 | | |
| Cyba | NP_000092.2 | Cyba | NP_001288213.1 |
| | XP_011521207.1 | | NP_031832.2 |
| Pld4 | NP_001295103.1 | Pld4 | NP_849242.1 |
| | NP_620145.2 | | |
| Tyrobp | NP_001166985.1 | Tyrobp | NP_035792.1 |
| | NP_001166986.1 | | |
| | NP_003323.1 | | |
| | NP_937758.1 | | |
| Cd84 | NP_001171808.1 | Cd84 | NP_001239401.1 |
| | NP_001171810.1 | | NP_001276399.1 |
| | NP_001171811.1 | | NP_038517.1 |
| | NP_001317671.1 | | |
| | NP_003865.1 | | |
| Emrl | NP_001243181.1 | Emrl | NP_001342651.1 |
| | NP_001243182.1 | | NP_001342652.1 |
| | NP_001243183.1 | | NP_034260.1 |
| | NP_001243184.1 | | |
| | NP_001965.3 | | |
| Csf3r | NP_000751.1 | Csf3r | NP_001239580.1 |
| | NP_724781.1 | | NP_031808.2 |
| | NP_758519.1 | | |

### [Table 8]

**Table 8: Further examples of nucleic acids encoding neuron-activating factors (3)**

| Human | | Mouse | |
|---|---|---|---|
| Name | NCBI Accession No. | Name | NCBI Accession No. |
| Lilrb4 | NM 001278426.3 | Lilrb4 | NM 001291894.1 |
| | NM 001278427.3 | | NM 013532.3 |
| | NM 001278428.3 | | |
| | NM 001278429.3 | | |
| | NM 001278430.3 | | |
| Clec7a | NM_022570.5 | Clec7a | NM 001309637.2 |
| | NM 197947.3 | | NM 020008.4 |
| | NM_197948.3 | | |
| | NM 197949.3 | | |
| | NM 197950.3 | | |
| | NM_197954.3 | | |
| Tlr2 | NM 001318787.2 | Tlr2 | NM 011905.3 |
| | NM 001318789.2 | | |
| | NM 001318790.2 | | |
| | NP_001305720.1 | | |
| | NM 001318793.2 | | |
| | NM 001318795.2 | | |
| | NM 001318796.2 | | |
| | NM 003264.5 | | |
| Gpnmb | NM_001005340.2 | Gpnmb | NM 053110.4 |
| | NM 002510.3 | | |
| C3ar1 | NM 001326475.2 | C3ar1 | NM 009779.2 |
| | NM 001326477.2 | | |
| | NM 004054.4 | | |
| Ecel1 | NM 001290787.2 | Ecel1 | NM 001277925.1 |
| | NM 004826.4 | | NM 021306.3 |
| Tmem106a | NM_001291586.2 | Tmem106a | NM 001359325.1 |
| | NM_001291587.2 | | NM 001359326.1 |
| | NM 001291588.2 | | NM 001359327.1 |
| | NM 145041.4 | | NM 144830.3 |
| Ms4a7 | NM 021201.5 | Ms4a7 | NM 001025610.4 |
| | NM_206938.2 | | NM 001276398.1 |
| | NM 206939.2 | | NM 027836.6 |
| | NM 206940.2 | | |
| Cd48 | NM_001256030.2 | Cd48 | NM_001360767.1 |
| | NM 001778.4 | | NM 007649.5 |
| Ifi30 | NM 006332.5 | Ifi30 | NM 023065.3 |
| Cd180 | \| NM 005582.3 | Cd180 | NM 001360519.1 |
| | | | NM_008533.2 |
| Fcgr2b | NM 001002273.3 | Fcgr2b | NM_001077189.1 |
| | NM 001002274.3 | | NM 010187.2 |
| | NM 001002275.3 | | |
| | NM 001190828.2 | | |
| | NM 001386000.1 | | |
| | NM 001386001.1 | | |
| | NM 001386002.1 | | |
| | NM 001386003.1 | | |
| | NM 001386004.1 | | |
| | NM 001386005.1 | | |
| | NM 001386006.1 | | |
| | NM 004001.5 | | |
| Hvcn 1 | NM 001040107.2 | Hvcn1 | NM 001042489.2 |
| | NM 001256413.2 | | NM 001359454.1 |
| | NM 032369.4 | | NM 028752.3 |
| Bst2 | NM 004335.4 | Bst2 | NM_198095.3 |
| Rtp4 | NM 022147.3 | Rtp4 | XM 036160102.1 |
| | | | XM_030249256.2 |
| | | | XM 006522491.5 |
| | | | XM 030249257.2 |
| Trem2 | NM_001271821.2 | Trem2 | NM_001272078.1 |
| | NM 018965.4 | | NM 031254.3 |
| Cd68 | NM_001040059.2 | Cd68 | NM 001291058.1 |
| | NM_001251.3 | | |
| Hk2 | NM 000189.5 | Hk2 | NM 013820.3 |
| | NM 001371525.1 | | |
| Kcne5 | NM 012282.4 | Kcnell | NM 021487.1 |
| Abca1 | NM 005502.4 | Abca1 | NM 013454.3 |
| Icam 1 | NM 000201.3 | Icam1 | NM 010493.3 |
| P2ry6 | NM 001277204.2 | P2ry6 | NM_183168.2 |
| | NM 001277205.2 | | XM_036152995.1 |
| | NM 001277206.2 | | XM_011241739.4 |
| | NM 001277207.2 | | XM 006507640.4 |
| | NM 001277208.1 | | |
| | NM 176796.3 | | |
| | NM 176797.3 | | |
| | NM 176798.2 | | |
| Tbxas1 | NM 001061.7 | Tbxas1 | NM_011539.3 |
| | NM 001130966.5 | | |
| | NM 001166253.4 | | |
| | NM_001166254.4 | | |
| | NM 001314028.4 | | |
| | NM 001366537.3 | | |
| | NM 001366538.2 | | |
| | NM 030984.6 | | |
| Slc37a2 | NM 001145290.2 | Slc37a2 | NM 001145960.1 |
| | NM 198277.3 | | NM 020258.4 |
| Hmox 1 | NM 002133.3 | Hmox1 | NM 010442.2 |
| Epsti1 | NM 001002264.4 | | NM_029495.2 |
| | NM 001330543.2 | Epsti1 | NM 178825.4 |
| | NM 001331228.2 | | |
| | NM_033255.5 | | |
| Slc15a3 | NM 016582.3 | Slc15a3 | NM 023044.2 |
| Cd74 | NM 001025158.2 | Cd74 | NM_001042605.1 |
| | NM 001025159.2 | | NM 010545.3 |
| | NM 001364083.2 | | |
| | NM 001364084.2 | | |
| | NM 004355.3 | | |
| Cyba | NM 000101.4 | Cyba | NM 001301284.1 |
| | XM 011522905.3 | | NM 007806.3 |
| Pld4 | NM 001308174.2 | Pld4 | NM 178911.4 |
| | NM_138790.5 | | |
| Tyrobp | NM 001173514.2 | Tyrobp | NM_011662.3 |
| | NM 001173515.2 | | |
| | NM 003332.4 | | |
| | NM 198125.3 | | |
| Cd84 | NM 001184879.2 | Cd84 | NM 001252472.1 |
| | NM 001184881.2 | | NM_001289470.1 |
| | NM 001184882.2 | | NM 013489.3 |
| | NM 001330742.2 | | |
| | NM 003874.4 | | |
| Emrl | NM 001256252.2 | Emrl | NM 001355722.1 |
| | NM 001256253.2 | | NM 001355723.1 |
| | NM 001256254.2 | | NM 010130.4 |
| | NM 001256255.2 | | |
| | NM 001974.5 | | |
| Csf3r | NM 000760.4 | Csf3r | NM 001252651.1 |
| | NM_156039.3 | | NM 007782.3 |
| | NM 172313.3 | | |

Amount of an active ingredient contained in a neuron-activating factor varies depending on the type or form of the active ingredient (a protein or a nucleic acid such as DNA or RNA), dosage form of the neuron-activating factor, and the type of solvent or carrier described below. Thus, it may be determined as appropriate based on respective conditions. Usually, an agent for activating a neuron is adjusted to contain an effective amount of an active ingredient in a single-dose. However, when it is necessary to administer a large amount of an agent for activating a neuron to a subject to achieve a pharmacological effect of the active ingredient, the agent for activating a neuron may be administered in several separate instances to reduce stress on the subject. In such a case, it is sufficient if the total amount contains an effective amount of the active ingredient.

The term "effective amount" used in the present aspect refers to an amount which is necessary to exert the function of an active ingredient and which produces almost no or no harmful side effect on the subject to which it is applied. Such effective amount can vary depending on various conditions, such as information on the subject, the route of administration, and the number of administration. When an agent for activating a neuron according to the present invention is used as a medicine, the amount of the contained active ingredieant is decided by a physician or a pharmacist in the end. For example, an agent for activating a neuron according to the present invention may be used in an amount of 0.01 ng to 10,000 ng, 0.1 ng to 1,000 ng, or 1 ng to 100 ng, *e.g.,* 1 ng, 10 ng, 20 ng, 50 ng, or 100 ng.

The term "subject" used herein refers to a living body to which an agent for activating a neuron according to the present invention is administered. Examples thereof include human, livestock animal (*e.g.,* cattle, horse, sheep, goat, pig, chicken, and ostrich), racehorse, pet animal (*e.g.,* dog, cat, and rabbit), and laboratory animal *(e.g.,* mouse, rat, guinee pig, monkey, and marmoset). A subject is preferably a human (a human subject is referred to as a "human subject" in particular). For example, a subject is a human who has a neural disease or a human who has a possibility of developing a neural disease.

The term "information on a subject" used herein refers to a variety of information concerning features and conditions of the subject. Examples thereof include age, body weight, gender, health conditions of whole body, the presence or absence of a disease, progression or severity of a disease, drug-sensitivity, the presence or absence of a drug to be used in combination, and resistance to treatment.

In an embodiment, activating a neuron is promotion of extension of an axons, an axon collateral, and/or a dendrite. In another embodiment, activating a neuron is suppression (reduction) of degeneration of a neuron and/or suppression of neuronal cell death.

### 1-3-2. Dosage form

A dosage form of an agent for activating a neuron according to the present invention is not particularly limited. The dosage form may be a form which can deliver the active ingredient to a site of interest without inactivation in the body of the subject.

A specific dosage form varies depending on the application method described below. An application method can be largely classified into parenteral administration and oral administration, and an appropriate dosage form may be employed in accordance with the respective method of administration.

If the method of administration is parenteral administration, a preferable dosage form is a liquid formulation that can be administered directly to the target site or administered systemically through the circulatory system. An example of a liquid formulation is an injection formulation. An injection formulation can be formulated by mixing as appropriate with an excipient, an emulsifier, a suspending agent, a surfactant, a stabilizer, a pH modifier, and the like to a unit dosage form required for generally approved pharmaceutical practice. In addition, for the purpose of controlled-release of an active ingredient or a local effect, it may be formulated as an artificial extracellular matrix that can be implanted into the living body.

If the method of administration is oral administration, examples of a preferable dosage form include solid medicine (including tablet, capsule, drop, and troche), granule, dust formulation, powder, and liquid formulation (including a mixture for internal use, an emulsion, and a syrup). Solid medicine can be prepared in the form that is coated with a coating material known in the art, as necessary, and examples thereof include sugar-coated tablet, gelatin-coated tablet, enteric tablet, film-coated tablet, double-coated tablet, and multilayered tablet.

The specific configuration and size of each dosage form described above are not particularly limited, provided that the configuration and size are within the range of dosage forms known in the art. Regarding a method of manufacturing an agent for activating a neuron of the present invention, it may be prepared in accordance with a conventional technique in the art.

### 1-3-3. Method of application

The application route of an agent for activating a neuron according to the present invention may be oral administration or parenteral administration. In general, a method of oral administration is systemic administration. A method of parenteral administration can be further classified into topical administration and systemic administration. Examples of topical administration include intracerebral administration and intraventricular administration. Examples of systemic administration include intravenous administration (IV) and intraarterial administration. When the agent for activating a neuron according to the present invention is to be administered topically, for example, it may be directly injected into the brain, such as cerebrum (e.g., frontal lobe and temporal lobe), brain stem, midbrain, or substantia nigra, retina, or eyeball, or may be administered into cerebral ventricle. In the case of systemic administration, IV administration may be employed. The dose is not particularly limited, provided that the active ingredient sufficiently produces an effect. An effective amount is selected as appropriate in accordance with the information on the subject as described above.

### 1-4. Effect

An agent for activating a neuron according to the present invention activates a neuron (*e.g.,* a brain neuron), providing an effect of promoting survival, differentiation, maturation, and the like of a neuron (*e.g.,* an effect of promoting extension of an axon or an effect of increasing the number of axon collaterals), and an effect of protecting a neuron from an injury (*e.g.,* an effect of suppressing neuronal cell death, or an effect of suppressing degeneration of a neuron). Accordingly, an agent for activating a neuron according to the present invention can be used as an agent for promoting neuronal maturation, an agent for promoting neuronal differentiation, an agent for promoting neurite extension (an agent for promoting extension of an axon, an axon collateral, and/or a dendrite), an agent for protecting a neuron, an agent for suppressing neuronal cell death, and/or an an agent for suppressing degeneration of a neuron.

### 2. An agent for assisting cell transplantation

### 2-1. Overview

The second aspect of the present invention is an agent for assisting cell transplantation. An agent for assisting cell transplantation of the present aspect comprises an agent for activating a neuron described in the first aspect. More specifically, an agent for assisting cell transplantation comprises a protein selected from the group consisting of proteins such as LYZ, CD52, MPEG1, CCL2, CST7, RAC2, SERPINA3, ANOS1, KLK6, A2M, GM14295, SPP1, LGALS3BP, APOC1, PGrn, Ctsd, Ctss, Apod, and Sparc, an active fragment of any of them, and an ortholog of any of them or a nucleic acid encoding said protein, and is used for the purpose of assisting cell transplantation.

### 2-2. Definition

The term "assisting cell transplantation" used herein refers to assistance of cell transplantation by promoting at least one of general evaluation criteria for cell transplantation, such as survival of an *in vivo* transplanted cell, differentiation into a desired cell, and the differentiation level. More specifically, assistance of cell transplantation may be based on increasing the number or density of desired cells derived from the transplanted cells, or promoting the maturity as the desired cells.

Examples of a "transplanted cell (cells for transplantation)" which can be preferably used herein include a neuron, and a cell which can differentiate into a neuron. Examples of a cell which can differentiate into a neuron include a neural stem cell and a neural progenitor cell. A transplanted cell may be a cell derived from a living body (a cell collected from a biological tissue of an animal of interest) or an artificially prepared cell. A transplanted cell preferably has a histocompatibility matching or within a range acceptable to a recipient. For example, a transplanted cell may be any of a cell of a recipient, a cell derived from a donor whose histocompatibility is within an acceptable range, or a cell prepared with a gene editing technique from a donor cell whose histocompatibility is out of an acceptable range.

The term "neural stem cell" used herein refers to a stem cell which has a pluripotency of differentiating into a neuron or a glial cell and has an ability of self-replication. In addition, the term "neural progenitor cell" refers to an undifferentiated cell having an ability of differentiating only to a neuron. The type of a neural stem cell or a neural progenitor cell is herein not particularly limited, and any neural stem cell or neural progenitor cell can be used, as long as it is capable of differentiating into a neuron of interest.

The term "pluripotent stem cell" used herein refers to a stem cell which has pluripotency of being capable of differentiating into a variety of cells existing *in vivo* and also additionally has an ability of proliferation. Examples thereof include, but are not limited to, an embryonic stem (ES) cell, an embryonic stem cell produced from a clone embryo via nuclear transfer (ntES cell), sperm stem cell (GS cell), embryonic germ cell (EG cell), induced pluripotent stem cell (iPS cell), and a pluripotent cell derived from a fibroblast or a myeloid stem cell (Muse cell).

The term "artificially produced cell" used herein refers to, for example, a cell produced from a pluripotent stem cell through induced differentiation, or a cell directly induced from a somatic cell via a direct reprogramming method without going through a pluripotent stem cell. A cell produced from a pluripotent stem cell through induced differentiation may be, for example, a cell of an organoid prepared from a human pluripotent stem cell.

The term "maturation of a neuron" used herein refers to, but is not limited to, for example, extension of a neurite (*e.g.,* an axon, an axon collateral, and/or a dendrite), formation and extension of a spine, and synapsis formation or circuit formation with other neurons or a target cell. Promotion of extension of an axon collateral or a dendrite include, for example, increase in the neurite length and promotion of branching.

### 2-3. Configuration

### 2-3-1. Component

An agent for assisting cell transplantation according to the present invention comprises an agent for activating a neuron described in the first aspect. More specifically, an agent for assisting cell transplantation according to the present invention comprises, as an essential active ingredient, a protein selected from the group consisting of each protein of LYZ, CD52, MPEG1, CCL2, CST7, RAC2, SERPINA3, ANOS1, KLK6, A2M, GM14295, SPP1, LGALS3BP, APOC1, PGrn, Ctsd, Ctss, Apod, and Sparc shown in Table 1 and Table 1', each protein shown in Tables 3, 5, 7, and 9, an active fragment of any of them, and an ortholog of any of them (herein referred to as "a transplantation-assisting factor"; a transplantation-assisting factor may be referred to as a neuron-activating factor as described in the first aspect above, and these terms can be used interchangeably herein) or a nucleic acid encoding said protein, said active fragment, or said ortholog (herein referred to as a "nucleic acid encoding a transplantation-assisting factor"; Examples thereof include the nucleic acids shown in Table 2, Table 2', Table 4, Table 6, and Table 8 described above. As described in the first aspect above, a nucleic acid encoding a transplantation-assisting factor can also be referred to as a nucleic acid encoding a neuron-activating factor, and these terms can be used interchangeably herein.) Each active ingredient is in accordance with the "neuron-activating factor" or the "nucleic acid encoding a neuron-activating factor" as described in the first aspect. For example, an agent for assisting cell transplantation according to the present invention comprises, as an essential active ingredient, a protein selected from the group consisting of each protein of LYZ, CST7, CCL12, CD52, MPEG1, RAC2, SPP1, PGrn, Ctsd, Ctss, Apod, and Sparc, an active fragment of any of them, and an ortholog of any of them or a nucleic acid encoding said protein, said active fragment, or said ortholog. In an embodiment, an agent for assisting cell transplantation according to the present invention comprises, as an essential active ingredient, a protein selected from the group consisting of each protein of LYZ, SPP1, PGrn, Ctsd, Ctss, Apod, and Sparc, an active fragment of any of them, and an ortholog of any of them or a nucleic acid encoding said protein, said active fragment, or said ortholog. In a preferable embodiment, an agent for assisting cell transplantation according to the present invention comprises, as an essential active ingredient, a protein selected from the group consisting of each protein of LYZ, SPP1, PGrn, and Sparc as described above, an active fragment of any of them, and an ortholog of any of them or a nucleic acid encoding said protein, said active fragment, or said ortholog. In a more preferable embodiment, an agent for assisting cell transplantation according to the present invention comprises, as an essential active ingredient, a protein selected from the group consisting of each protein of LYZ, SPP1, and PGrn as described above, an active fragment of any of them, and an ortholog of any of them or a nucleic acid encoding said protein, said active fragment, or said ortholog. In a furthermore preferable embodiment, an agent for assisting cell transplantation according to the present invention comprises, as an essential active ingredient, a protein selected from the group consisting of each protein of LYZ or PGrn as described above, an active fragment of any of them, and an ortholog of any of them or a nucleic acid encoding said protein, said active fragment, or said ortholog. In a particularly preferable embodiment, an agent for assisting cell transplantation according to the present invention comprises, as an essential active ingredient, a protein selected from the group consisting of LYZ described above, an active fragment thereof, and an ortholog thereof or a nucleic acid encoding said LYZ, said active fragment, or said ortholog. Since each active ingredient is in accordance with description in the first aspect, further description thereof is omitted here.

Examples of transplantation-assisting factors, NCBI accession numbers thereof, and SEQ ID NOs thereof are in accordance with neuron-activating factors exemplified in Table 1 and Table 1' described above, and description thereof is accordingly omitted here.

A transplantation-assisting factor may be introduced into a transplanted cell or a cell differentiated from a transplanted cell using a technique such as lipofection, fusion with a cell-penetrating peptide (e.g., HIV-derived TAT, polyarginine, BP100, Penetratin, pVEC, pAntp, VP22, Transportan, R7, MPG, and Pep-1), or microinjection.

The term "a nucleic acid encoding said protein, said active fragment, or said ortholog" (also referred to as "a nucleic acid encoding a transplantation-assisting factor") used herein refers to a nucleic acid encoding any of the transplantation-assisting factors described above. A nucleic acid encoding a transplantation-assisting factor encodes LYZ, CD52, MPEG1, CCL2, CST7, RAC2, SERPINA3, ANOS1, KLK6, A2M, GM14295, SPP1, LGALS3BP, APOC1, PGrn, Ctsd, Ctss, Apod, or Sparc, a mutant protein described above derived from any of them, an active fragment of any of them, or an ortholog described above of any of them, regardless of whether it is DNA or RNA. DNA encoding a transplantation-assisting factor may be a gene encoding a transplantation-assisting factor or cDNA produced from the transcription product thereof as a template. RNA encoding a transplantation-assisting factor encompasses both mRNA precursor (pre-mRNA) and mature mRNA, but substantially means mature mRNA.

Specific examples of DNA encoding a transplantation-assisting factor (cDNA encoding a transplantation-assisting factor) include DNA encoding a human wild-type LYZ protein, which consists of the base sequence as shown in SEQ ID NO: 46; DNA encoding a mouse wild-type LYZ2 protein, which consists of the base sequence as shown in SEQ ID NO: 45; DNA encoding ahuman wild-type CD52 protein, which consists of the base sequence as shown in SEQ ID NO: 48; DNA encoding amouse wild-type CD52 protein, which consists of the base sequence as shown in SEQ ID NO: 47; DNA encoding a human wild-type MPEG1 protein, which consists of the base sequence as shown in SEQ ID NO: 51; DNA encoding a mouse wild-type MPEG1 protein, which consists of the base sequence as shown in SEQ ID NO: 49 or 50; DNA encoding a human wild-type CCL2 protein, which consists of the base sequence as shown in SEQ ID NO: 53; DNA encoding a mouse wild-type CCL12 protein, which consists of the base sequence as shown in SEQ ID NO: 52; DNA encoding a human wild-type CST7 protein, which consists of the base sequence as shown in SEQ ID NO: 55; DNA encoding a mouse wild-type CST7 protein, which consists of the base sequence as shown in SEQ ID NO: 54; DNA encoding a human wild-type RAC2 protein, which consists of the base sequence as shown in SEQ ID NO: 57; DNA encoding a mouse wild-type RAC2 protein, which consists of the base sequence as shown in SEQ ID NO: 56; DNA encoding a human wild-type SERPINA3 protein, which consists of the base sequence as shown in SEQ ID NO: 59; DNA encoding a mouse wild-type SERPINA3N protein, which consists of the base sequence as shown in SEQ ID NO: 58; DNA encoding a human wild-type ANOS1 protein, which consists of the base sequence as shown in SEQ ID NO: 61; DNA encoding a mouse wild-type GM11428 protein, which consists of the base sequence as shown in SEQ ID NO: 60; DNA encoding a human wild-type KLK6 protein, which consists of the base sequence as shown in any of SEQ ID NOs: 63 to 67; DNA encoding a mouse wild-type KLK6 protein, which consists of the base sequence as shown in SEQ ID NO: 62; DNA encoding a human wild-type A2M protein, which consists of the base sequence as shown in any of SEQ ID NOs: 69 to 72; DNA encoding a mouse wild-type A2M protein, which consists of the base sequence as shown in SEQ ID NO: 68; DNA encoding a mouse wild-type GM14295 protein, which consists of the base sequence as shown in SEQ ID NO: 73; DNA encoding a human wild-type SPP1 protein, which consists of the base sequence as shown in SEQ ID NO: 79 or 80; DNA encoding a mouse wild-type SPP1 protein, which consists of the base sequence as shown in any of SEQ ID NOs: 74 to 78; DNA encoding a human wild-type LGALS3BP protein, which consists of the base sequence as shown in SEQ ID NO: 82; DNA encoding a mouse wild-type LGALS3BP protein, which consists of the base sequence as shown in SEQ ID NO: 81; DNA encoding a human wild-type APOC1 protein, which consists of the base sequence as shown in any of SEQ ID NOs: 85 to 88; DNA encoding a mouse wild-type APOC1 protein, which consists of the base sequence as shown in SEQ ID NO: 83 or 84 protein; DNA encoding a human wild-type PGrn protein, which consists of the base sequence as shown in SEQ ID NO: 107; DNA encoding a mouse wild-type PGrn protein, which consists of the base sequence as shown in SEQ ID NO: 106; DNA encoding a human wild-type Ctsd protein, which consists of the base sequence as shown in SEQ ID NO: 109; DNA encoding a mouse wild-type Ctsd protein, which consists of the base sequence as shown in SEQ ID NO: 108; DNA encoding a human wild-type Ctss protein, which consists of the base sequence as shown in SEQ ID NO: 112 or 113; DNA encoding a mouse wild-type Ctss protein, which consists of the base sequence as shown in SEQ ID NO: 110 or 111; DNA encoding a human wild-type Apod protein, which consists of the base sequence as shown in SEQ ID NO: 117; DNA encoding a mouse wild-type Apod protein, which consists of the base sequence as shown in any of SEQ ID NOs: 114 to 116; DNA encoding a human wild-type Sparc protein, which consists of the base sequence as shown in any of SEQ ID NOs: 120 to 122; and DNA encoding a mouse wild-type Sparc protein, which consists of the base sequence as shown in SEQ ID NO: 118 or 119.

Further, an example of mRNA encoding a transplantation-assisting factor is mRNA comprising, as a coding region (translated region), a base sequence derived from any of the base sequences described above in which t (thymine) is substituted with u (uracil). mRNA encoding a transplantation-assisting factor may comprise, in addition to said coding region, a 5'-terminal cap structure, a 3'-terminal poly A chain, a 5' untranslated region (5' UTR) upstream of the start codon, and/or a 3' untranslated region (3' UTR) downstream of the stop codon. 5' UTR and/or 3' UTR may comprise a sequence for regulating the amount of translation from mRNA. For example, 3' UTR may comprise a sequence for increasing the amount of translation from mRNA (e.g., the woodchuck hepatitis virus post-transcriptional regulatory element (WPRE)).

Examples of nucleic acids encoding a transplantation-assisting factor, NCBI accession numbers corresponding thereto, and SEQ ID NOs are in accordance with the nucleic acids encoding a neuron activating factor exemplified in Table 2 and Table 2' above, and description thereof is accordingly omitted here.

In an agent for assisting cell transplantation according to the present invention, a nucleic acid encoding a transplantation-assisting factor may be DNA or RNA (e.g., mRNA). When a nucleic acid is in the form of DNA, it may be contained in a gene expression vector. A nucleic acid may be introduced into a transplanted cell or a cell differentiated from a transplanted cell by means of, for example, electroporation, lipofection, liposome, or microinjection.

"A state which allows for expression" in the present aspect means that a gene or the like encoding a transplantation-assisting factor is positioned in a downstream region, which is under the control of a promoter. Accordingly, in a cell into which a vector is introduced (e.g., a transplanted cell), expression of a transplantation-assisting factor is induced by a promoter.

In the present aspect, various gene expression vectors which can replicate and allow for expression in a transplanted cell or a cell differentiated from a transplanted cell (e.g., a neuron) can be used. Examples thereof include a viral vector, a plasmid vector, and an artificial chromosome vector. A virus vector is not particularly limited, provided that it can infect a cell (e.g., a cell in the brain) and express a transplantation-assisting factor in the same cell. Examples thereof include adenovirus vector, adeno-associated virus (AAV) vector, retrovirus vector, lentivirus vector, and Sendai virus vector. A plasmid vector is not particularly limited, provided that it can replicate in a mammalian cell. Specific examples of a plasmid vector include pCMV6-XL3, pEGFP-C1, pGBT-9, pcDNA, pcDM8, and pREP4. Examples of an artificial chromosome vector include human artificial chromosome (HAC) vector, yeast artificial chromosome (YAC) vector, and bacterial artificial chromosome (BAC or PAC).

In the present aspect, a promoter is a promoter having an activity of inducing gene expression in a transplanted cell or a cell differentiated from a transplanted cell (*e.g.,* a neuron). Examples thereof include CMV promoter (CMV-IE promoter), LTR promoter, SV40 promoter, RSV promoter, HSV-TK promoter, EF1α promoter, Ub promoter, metallothionein promoter, SRα promoter, and CAG promoter.

When a nucleic acid is in the form of RNA, it may be introduced into a cell by means of, for example, electroporation, lipofection, liposome, or microinjection.

In an embodiment, an agent for assisting cell transplantation may comprise multiple types of or multiple forms of active ingredients.

In another aspect, a transplantation-assisting factor used in an agent for assisting cell transplantation according to the present invention may be a protein selected from the group consisting of any of the neuron activating factors shown in Tables 3, 5, 7, and 9 above, an active fragment of any of them, and an ortholog of any of them. Nucleic acids encoding a transplantation-assisting factor corresponding to the neuron-activating factor shown in Tables 3, 5, and 7, and NCBI accession numbers corresponding thereto are in accordance with the nucleic acids encoding the neuron-activating factors exemplified in Tables 4, 6, and 8 above, and description thereof is accordingly omitted here.

The amount of an active ingredient to be contained in an agent for assisting cell transplantation varies depending on the type or form of the active ingredient (a protein, or a nucleic acid such as DNA or RNA), a dosage form of the agent for assisting cell transplantation, and the type of solvent or a carrier described below. Thus, it may be determined as appropriate based on repective conditions. Usually, an agent for assisting cell transplantation is adjusted to contain an effective amount of an active ingredient in a single dose of the agent for assisting cell transplantation. However, when it is necessary to administer a large amount of an agent for assisting cell transplantation to a subject to achieve a pharmacological effect of an active ingredient, the agent for assisting cell transplantation may be administered in several separate instances to reduce stress on the subject. In such a case, it is sufficient if the total dose of administration contains an effective amount of the active ingredient.

The term "effective amount" used in the present aspect refers to an amount which is necessary for exerting a function of an active ingredient and which produces almost no or no harmful side effect on the subject to which it is applied. This effective amount can vary depending on various conditions such as information on the subject, the route of administration, and the number of administration. When an agent for assisting cell transplantation according to the present invention is used as a medicine, the amount of the contained active ingredient is decided by physicians or pharmacists in the end. For example, for 100,000 transplanted cells, an agent for assisting cell transplantation according to the present invention may be used in an amount of 0.01 ng to 10,000 ng, 0.1 ng to 1,000 ng, or 1 ng to 100 ng, *e.g.,* 1 ng, 10 ng, 20 ng, 50 ng, or 100 ng.

The term "subject" used in the present aspect refers to a living body to which an agent for assisting cell transplantation according to the present invention is applied. Examples thereof include human, livestock animal (*e.g.,* cattle, horse, sheep, goat, pig, chicken, and ostrich), racehorse, pet animal (*e.g.,* dog, cat, and rabbit), and laboratory animal (*e.g.,* mouse, rat, guinee pig, monkey, and marmoset). A subject is preferably a human (a human subject is referred to as a "human subject," in particular). For example, a subject is a human who has a neural disease or a human who has a possibility of developing a neural disease. In particular, a subject who is subjected to cell transplantation is referred to as a "recipient."

### 2-3-2. Dosage form

A dosage form of an agent for assisting cell transplantation according to the present invention is not particularly limited. The dosage form may be a form which can deliver the active ingredient to a site of interest such as a transplanted site or a neural injury site without inactivation in the body of the subject.

A specific dosage form varies depending on the application method described below. An application method can be largely classified into parenteral administration and oral administration, and an appropriate dosage form may be employed in accordance with the respective method of administration.

If the method of administration is parenteral administration, a preferable dosage form is a liquid formulation that can be administered directly to the target site such as a transplanted site or a neural injury site or administered systemically through the circulatory system. An example of a liquid formulation is an injection formulation. An injection formulation can be formulated by mixing as appropriate with said excipient, an emulsifier, a suspending agent, a surfactant, a stabilizer, a pH modifier, and the like to a unit dosage form required for generally approved pharmaceutical practice. In addition, for the purpose of controlled-release of an active ingredient or a local effect, it may be formulated as an artificial extracellular matrix that can be implanted into the living body.

If the method of administration is oral administration, examples of a preferable dosage form include solid medicine (including tablet, capsule, drop, and troche), granule, dust formulation, powder, and liquid formulation (including a mixture for internal use, an emulsion, and a syrup). Solid medicine can be prepared in the form that is coated with a coating material known in the art, as necessary, and examples thereof include sugar-coated tablet, gelatin-coated tablet, enteric tablet, film-coated tablet, double-coated tablet, and multilayered tablet.

The specific configuration and size of each dosage form described above are not particularly limited, provided that the configuration and size are within the range of dosage forms known in the art. Regarding a method of manufacturing an agent for activating a neuron of the present invention, it may be prepared in accordance with a conventional technique in the art.

### 2-3-3. Method of application

The application route of an agent for assisting cell transplantation according to the present invention may be oral administration or parenteral administration. In general, a method of oral administration is systemic administration. A method of parenteral administration can be further classified into topical administration and systemic administration. Examples of topical administration include, *e.g.*, intracerebral administration and intraventricular administration, administration to the site of transplantation of the transplanted cell or an area in the vicinity thereof, and administration to a site where the cell body of the neuron projecting to the transplanted site is present. Examples of a transplanted site are the sites exemplified in the section of "a brain neuron" above. Examples of systemic administration include intravenous administration (IV) and intraarterial administration. The dose may be those by which the active ingredient sufficiently produces an effect. An effective amount is selected as appropriate in accordance with the information on the subject as described above.

The timing of administration of an agent for assisting cell transplantation according to the present invention is not limited, and may be before cell transplantation, simultaneous with cell transplantation, after cell transplantation, or a combination of them. The term "cell transplantation" may refer to "a procedure of introducing a cell for transplantation into the body of a recipient." The phrase "simultaneous with cell transplantation" means "the same day with the day of cell transplantation," and preferably "from the initiation to the completion of the step for cell transplantation." For example, an agent for assisting cell transplantation according to the present invention may be administered to a recipient within 6 hours, 4 hours, 2 hours, or 1 hour before or after cell transplantation. The phrase "before cell transplantation" includes an aspect in which a cell for transplantation is treated with an agent for assisting cell transplantation in advance, and the cell for transplantation after the treatment is then transplanted. In a preferable embodiment, a cell for transplantation may be treated with an agent for cell transplantation according to the present invention before cell transplantation. For example, a cell for transplantation may be cultured in the presence of an agent for assisting cell transplantation according to the present invention for a certain period of time before cell transplantation. The period of culturing in the presence of an agent for assisting cell transplantation according to the present invention is not limited. For example, it may be 2 hours or longer, 12 hours or longer, 1 day or longer, 2 days or longer, 3 days or longer, 4 days or longer, 1 week or longer, or 2 weeks or longer. The culture condition before cell transplantation is not limited. For example, for 100,000 cells for transplantation, a cell for transplantation may be cultured in a culture liquid containing an agent for assisting cell transplantation in an amount of 0.01 ng to 10,000 ng, 0.1 ng to 1,000 ng, or 1 ng to 100 ng, *e.g.,* 1 ng, 10 ng, 20 ng, 50 ng, or 100 ng.

In an embodiment, an agent for assisting cell transplantation according to the present invention may be administered to a recipient simultaneously with transplantation to the recipient of a cell for transplantation which has been treated with an agent for assisting cell transplantation according to the present invention before cell transplantation.

The number of administration of an agent for assisting cell transplantation according to the present invention is not limited, and administration may be performed in a single instance or in a plurality of instances. For example, when an agent for assisting cell transplantation according to the present invention is administered after cell transplantation, administration may be performed in a single instance or in a plurality of instances at any timing after transplantation.

When an agent for assisting cell transplantation according to the present invention is administered after cell transplantation, it is preferable that the agent for assisting cell transplantation according to the present invention may be continuously infused. For example, an agent for assisting cell transplantation according to the present invention may be administered to a recipient at least once every 2 weeks, at least once every week, at least once every 2 days, at least once a day, or at least twice a day, after cell transplantation.

In an embodiment, an agent for assisting cell transplantation according to the present invention can be used in a mixture with a transplanted cell. For example, an agent for assisting cell transplantation according to the present invention may be mixed as a protein or nucleic acid in a cell suspension or cell mass containing a transplanted cell. When an agent for assisting cell transplantation according to the present invention is mixed as a protein with a cell for transplantation, the protein may be added directly or as a protein-embedded controlled-release matrix in a cell suspension or cell mass. Examples of controlled-release matrix include, but are not limited to, a naturally-occurring material such as an extracellular matrix (*e.g.,* collagen) and a biocompatible synthetic material.

The mixing ratio of an agent for assisting cell transplantation according to the present invention and a transplanted cell is not limited, provided that an effect on the transplanted cell is maintained. For example, for 100,000 transplanted cells, an agent for assisting cell transplantation according to the present invention can be mixed in an amount of 0.01 ng to 10,000 ng, 0.1 ng to 1,000 ng, or 1 ng to 100 ng, *e.g.,* 1 ng, 10 ng, 20 ng, 50 ng, or 100 ng.

In an embodiment, an agent for assisting cell transplantation according to the present invention may be introduced into a transplanted cell before cell transplantation and used, so that the transplanted cell itself can transiently express or maintain expression of the agent for assisting cell transplantation. For example, the transplantation-assisting factor may be introduced as a protein into a transplanted cell in accordance with a technique such as lipofection, fusion with a cell-penetrating peptide, or microinjection. Additionally, a transplantation-assisting factor may be introduced as a nucleic acid into a transplanted cell in accordance with a technique such as electroporation, lipofection, liposome, or microinjection.

### 3. Agent for treating or preventing neural disease

### 3-1. Overview

The third aspect of the present invention is an agent for treating or preventing a neural disease (hereafter, referred to as a "treating/preventing agent"). A treating/preventing agent of the present embodiment comprises an agent for activating a neuron of the first embodiment, and enables treatment or prevention of a neural disease such as neurodegenerative disease, head trauma, and cerebral infarction.

### 3-2. Configuration

### 3-2-1. Component

The treating/preventing agent according to the present invention can comprise, as an essential ingredient, an active ingredient, and, as an optional ingredient, a solvent, a pharmacologically acceptable carrier, or a further drug. A treating/preventing agent according to the present invention may be composed only of an active ingredient. However, in order to facilitate formation of a dosage form and maintain a pharmacological effect of an active ingredient and/or dosage form, however, it is preferable that the treating/preventing agent according to the present invention is configured as a medicine encompassing a pharmacologically acceptable carrier described below.

An active ingredient of a treating/preventing agent according to the present invention is an agent for activating a neuron according to the present invention, and the configuration thereof is described in detail in the first aspect. Accordingly, optional ingredients are only described below.

### (Solvent)

The treating/preventing agent according to the present invention can be dissolved in a pharmacologically acceptable solvent, as necessary. A "pharmacologically acceptable solvent" refers to a solvent which is usually used in the technical field of drug formulation. Examples thereof include water, an aqueous solution, and an organic solvent. Examples of aqueous solution include physiological saline, isotonic solution containing dextrose or other supplements, phosphate salt buffer, and sodium acetate buffer. Examples of supplements include D-sorbitol, D-mannose, D-mannitol, sodium chloride, and additionally, low-concentration nonionic surfactant, and polyoxyethylene sorbitan fatty acid ester. An example of an organic solvent is ethanol.

### (Carrier)

A treating/preventing agent according to the present invention can comprise a pharmacologically acceptable carrier, as necessary. A "pharmacologically acceptable carrier" is an additive which is usually used in the technical field of drug formulation. Examples thereof include excipient, binder, disintegrator, filler, emulsifier, flow modifier, lubricant, and human serum albumin.

Examples of excipient include sugar, such as monosaccharide, disaccharide, cyclodextrin, and polysaccharide, metal salt, citric acid, tartaric acid, glycine, polyethylene glycol, pluronic, kaolin, and silicic acid, and a combination thereof.

Examples of binder include starch glue derived from vegetable starch, pectin, xanthane gum, simple syrup, glucose liquid, gelatin, gum tragacanth, methyl cellulose, hydroxypropyl methyl cellulose, carboxylmethyl cellulose sodium, shellac, paraffin, and polyvinyl pyrrolidone, and a combination thereof.

Examples of disintegrator include said starch, lactose, carboxymethyl starch, crosslinked polyvinyl pyrrolidone, agar, laminaran powder, sodium bicarbonate, calcium carbonate, alginic acid or sodium alginate, polyoxyethylene sorbitan fatty acid ester, sodium lauryl sulfate, monoglyceride stearate, and a salt thereof.

Examples of filler include vaseline, sugar described above, and/or calcium phosphate.

Examples of emulsifier include sorbitan fatty acid ester, glycerin fatty acid ester, sucrose fatty acid ester, and propylene glycol fatty acid ester.

Examples of flow modifier and lubricant include silicate, talc, stearate, and polyethylene glycol.

In addition to those described above, a treating/preventing agent according to the present invention can comprise as appropriate solubilizer, suspending agent, diluent, dispersant, surfactant, soothing agent, stabilizer, absorption promoter, thickener, humidifier, moisturizer, wetting agent, adsorber, corrigent, disintegration inhibitor, coating agent, colorant, preservative, antiseptic agent, antioxidant, aroma chemical, flavoring agent, sweetener, buffer, isotonic agent, or the like which is usually used in a pharmaceutical composition or the like as necessary.

A carrier is used to avoid or suppress degradation of the above-described ingredient by enzyme or the like in the body of a subject, to facilitate drug formulation or administration, and to maintain dosage form and drug efficacy, and may be used as appropriate as necessary.

The dosage form and method of application of a treating/preventing agent according to the present invention are in accordance with the first aspect, and description thereof is omitted here.

### 3-2-2. Target disease

A target neural disease of a treating/preventing agent according to the present invention is not particularly limited, as long as involving degeneration, loss, or cell death of a neuron. Examples include neurodegenerative disease, brain tumor, head trauma, cerebral contusion, ischemia, cerebrovascular disorder, epilepsia, and multiple sclerosis.

A neurodegenerative disease is a disease in which a structure or function of a neuron is progressively lost with lesions. A neurodegenerative disease may be a disease involving accumulation of abnormal proteins in the brain or neuronal cell death. Specific neurodegenerative diseases include, but are not limited to, amyotrophic lateral sclerosis, primary lateral sclerosis, Parkinson's disease, FTDP-17, progressive supranuclear palsy, corticobasal degeneration, Huntington's disease, dystonia, neurodegeneration with iron accumulation in the brain, frontotemporal lobar degeneration, idiopathic calcification of the basal ganglia, essential tremor, Perry syndrome, mild cognitive impairment (MCI), Alzheimer's disease, argyrophilic grain disease, dementia with Lewy bodies, Down's syndrome, multiple system atrophy, degeneration of the cerebral cortex, prion disease, pantothenate kinase-associated neurodegeneration, dementia pugilistica, and chronic traumatic encephalopathy. A target disease of a treating/preventing agent according to the present invention is preferably Parkinson's disease, Alzheimer's disease, Huntington's disease, amyotrophic lateral sclerosis, dementia with Lewy bodies, spinocerebellar degeneration, progressive supranuclear palsy, and corticobasal degeneration.

Examples of ischemia (ischemic disease) include cerebrovascular dementia, cerebrovascular Parkinson's syndrome, cerebral amyloid angiopathy, ischemic optic nerve disorder, cerebrovascular disorder described below, and other diseases involving ischemic condition (e.g., cerebral meningitis, cerebritis, and cerebral abscess).

Examples of a cerebrovascular disorder includes cerebral stroke, cerebral infarction, infarction of spinal cord, cerebral venous sinus thrombosis, cerebral vasoconstriction syndrome, cerebral vasculitis, brain hemorrhage, subarachnoid hemorrhage, Moyamoya disease, cerebral arteriovenous fistulae, cerebral arteriovenous malformation, cerebral aneurysm, and cervical/cerebral artery dissection.

A target neural disease of a treating/preventing agent according to the present invention is preferably neurodegenerative disease, brain tumor, multiple sclerosis, epilepsia, head trauma, cerebral infarction, cerebral stroke, or ischemia.

### 3-3. Effects

The treating/preventing agent according to the present invention enables treatment or prevention of a neural disease through activation of a neuron such as a brain neuron, and promoting survival, differentiation, maturation, and the like thereof, and/or protecting a neuron.

### 4. Composition for treating neural disease

### 4-1. Overview

The fourth aspect of the present invention is a composition for treating a neural disease (hereafter simply referred to as a "composition for treatment"). The composition for treatment of the present aspect comprises an agent for assisting cell transplantation of the aspect and a cell for transplantation, and can be used for the purpose of treating a neural disease.

### 4-2. Configuration

### 4-2-1. Component

A composition for treatment according to the present invention can encompass, as an essential ingredient, an active ingredient, and, as an optional ingredient, a solvent, a pharmacologically acceptable carrier, or further drug. A composition for treatment according to the present invention may be composed only of an active ingredient. However, in order to facilitate formation of a dosage form and maintain a pharmacological effect of the active ingredient and/or dosage form, it is preferably configured as a medicine encompassing a pharmacologically acceptable carrier described below.

An active ingredient of the composition for treatment according to the present invention is an agent for assisting cell transplantation according to the present invention and a cell for transplantation (a transplanted cell). The configuration of an agent for assisting cell transplantation and a transplanted cell is already described in detail in the second aspect. Accordingly, only optional ingredients are described below.

### (Solvent)

The composition for treatment according to the present invention can be dissolved in a pharmacologically acceptable solvent as necessary. A "pharmacologically acceptable solvent" is in accordance with descriptions in the third aspect.

### (Carrier)

The composition for treatment according to the present invention can comprise a pharmacologically acceptable carrier, as necessary. A "pharmacologically acceptable carrier" is in accordance with description in the third aspect.

The dosage form and the method of application of the composition for treatment according to the present invention are in accordance with the second aspect, and description thereof is thus omitted here.

### 4-2-2. Target disease

A target neural disease of the composition for treatment according to the present invention is not particularly limited, as long as involving degeneration, loss, or neuronal cell death. A target neural disease of a composition for treatment according to the present invention is in accordance with the third aspect, and description thereof is omitted here.

The present invention provides a kit comprising an agent for activating a neuron described above. Further, the present invention also provides a kit comprising an agent for assisting cell transplantation according to the present invention and a cell for transplantation.

The present invention provides a method for treating or preventing a neural disease comprising a step of administering an agent for activating a neuron described above to a subject. Additionally, the present invention also provides a method for transplanting a cell to a recipient, comprising an administration step of administering an agent for assisting cell transplantation described above to a recipient, and a cell transplantation step transplanting a cell to the recipient.

The present invention provides a method for treating or preventing a neural disease comprising a step of administering an agent for activating a neuron described above to a subject. Additionally, the present invention provides use of an agent for assisting cell transplantation in the manufacture of a medicine for treating a neural disease.

### Examples

### <Example 1: Cell transplantation into a model of cerebral cortical injury>

### (Purpose)

Cell transplantation into the brain is carried out immediately after or 7 days after cortical injury, and the efficiency of survival of the transplanted cells and extension of axons are compared. It is examined how transplantation results change with differences in host brain environments.

### (Method and result)

### (1) Production of cell aggregates derived from the cerebral cortex tissue

The cerebral cortex tissue was isolated from the brain of a GFP mouse (C57BL/6-Tg (CAG-EGFP), Shimizu Laboratory Supplies Co., Ltd.) on embryonic day 13.5, and only live cells were separated using a cell sorter. The obtained live cells were cultured in a DMEM/F12 medium (DMEM/F12 supplemented with L-glutamine (2 mM), 2-mercaptoethanol (0.1 mM), B27-supplement (1×), N2-supplement (1×), penicillin/streptomycin (1×), Y-27632 (30 µM)) for 1 day. The cell aggregates obtained after the culture are shown in Fig. 1.

### (2) Transplantation of cell aggregates into a model of cortical injury

Models of cortical injury were prepared using postnatal 11-week-old immunodeficient mice (C.B-17/Icr-scid/scidJcl, CLEA Japan, Inc.). An injury was made at a depth of 1 mm from the brain surface at a position 2.0 mm rostal and lateral from the bregma using a biopsy trepan and an aspirator.

Immediately after or 7 days after cortical injury, 70 cell aggregates of above (1) were transplanted into the site of injury. Two months after transplantation, the mouse models were subjected to perfusion fixation, and frozen brain sections with a thickness of 30 µm were prepared. The brain sections were immunostained with the anti-GFP antibody (#04404-26, Nacalai tesque, INC.), and the distribution of the cells derived from the transplanted cell aggregates was observed. As a result, almost no survival of GFP-positive transplanted cells was observed in the case of cell transplantation immediately after cortical injury (Fig. 2A). In contrast, in the case of cell transplantation 7 days after cortical injury, survival of the transplanted cells and the promotion of extension of axons were observed (Fig. 2B).

In order to quantitatively compare the results of immunohistochemical staining shown in Fig. 2, the GFP immunostaining signals (fluorescence intensity) in the striatum were converted into numerical values using In cell analyzer. As a result, it was shown that the distribution of axons derived from the GFP-positive transplants was increased in the ipsilateral striatum in the group subjected to transplantation 7 days after cortical injury (Fig. 3).

### (3) Examination of corticospinal projection

It was investigated whether or not the axons of neurons derived from the transplanted cells are capable of corticospinal projection.

In some of the mice subjected to cell transplantation 7 days after cortical injury in above (2), a retrograde labeling reagent, Fast blue (#17740, Polysciences), was administered at the concentration of 4% to the first cervical nerve (C1) seven days before perfusion fixation (0.3 µl × 16 sites, 4.8 µl in total). In the frozen brain sections after perfusion fixation, it was investigated whether or not the GFP-positive cell bodies in the transplants were retrogradely labeled by Fast blue. As a result, GFP-positive and Fast blue-positive cell bodies were observed in the transplants (Fig. 4, arrowheads). The results demonstrated that neurons derived from the transplanted cells are capable of extending and projecting neuronal axons to C1 region.

### <Example 2: Gene expression analysis in the model of cerebral cortical injury 1>

### (Purpose)

Gene expression in the brain immediately after cortical injury (Day 0) or 7 days after cortical injury is compared.

### (Method and result)

By the same method as in Example 1, models of cortical injury were prepared using C57BL/6NCrSlc mice (Shimizu Laboratory Supplies Co., Ltd.). Immediately after cortical injury (Day 0, 5 to 10 minutes after injury) or 7 days after cortical injury, an area approximately 0.5 mm around and containing the site of cortical injury was isolated, and gene expression in the mouse cerebral cortex cells was analyzed by CAGE analysis. Immediately after cortical injury (Day 0) and 7 days thereafter, 5 mice were used, respectively. Screening was performed based on the criteria (1) or (2) described below, and candidate factors were extracted.

### (1) Factors whose gene expression was high 7 days after cortical injury

Based on the results of gene expression immediately after cortical injury (Day 0) and 7 days thereafter, candidate factors whose gene expression was high 7 days after cortical injury were extracted using the following four criteria (a) to (d):
(a) Log Fold change (FC) of 3.0 or more between Day 0 vs. Day 7;
(b) p value ≤ 0.05, and CPM value of 15 or higher on Day 7;
(c) Predicted to have a signal sequence using at least one of SignalP or SecretomeP, and the sequence is predicted to be positioned extracellularly using WoLF PSORT; and
(d) Functions of the gene are not related to inflammation, or apoptosis or phagocytosis and the like mediated by immune system, based on literatures.

Additionally, the "CPM (count-per-million) value" described above indicates the total read number of each gene. The "Log Fold-change (FC)" indicates a logarithmic change in the average count value on Day 7 relative to the average count value on Day 0. The "p value ≤ 0.05" indicates that there was a significant difference in CPM values between Day 0 and Day 7 by analyses independently performed 5 times.

As a result of screening based on the above criteria, 13 factors of Cst7, Spp1, A2m, Apoc1, Ccl12, Lyz2, Klk6, Gm11428, Cd52, Lgals3bp, Mpeg1, Rac2, and Serpina3n, were extracted (Fig. 5).

### (2) Factors whose gene expression was low 7 days after cortical injury

Based on the results of gene expression immediately after cortical injury (Day 0) and 7 days thereafter, candidate factors whose gene expression was low 7 days after cortical injury were extracted using the following four criteria (e) to (h):
(e) Log FC of -2.0 or less between Day 0 vs. Day 7;
(f) p value ≤ 0.05 and CPM value of 15 or higher on Day 0;
(g) Predicted to have a signal sequence using at least one of SignalP or SecretomeP, and the sequence is predicted to be positioned extracellularly using WoLF PSORT; and
(h) Functions of the gene are not related to inflammation, or apoptosis or phagocytosis and the like mediated by immune system, based on literatures.

As a result of screening based on the above criteria, Gm14295 was extracted (Fig. 5).

### <Example 3: Gene expression analysis in the model of cortical injury 2>

### (Purpose)

Based on the results of gene expression analysis in Example 2, further candidate factors are identified.

### (Method)

Gene/protein classification was performed using the following database: The human secretome and membrane proteome (https://www.proteinatlas.org/humanproteome/tissue/secretome). As a result, membrane proteins and secretory factors (116 genes), secretory factors (772 genes), and membrane proteins (3,422 genes) were extracted. The "membrane proteins and secretory factors" correspond to genes encoding a plurality of protein isoforms (splice variants) including secretory isoforms and membrane-bound isoforms.

A population of the extracted factors were subjected to screening for factors whose gene expression was high 7 days after cortical injury, based on the results of gene expression analysis of Example 2. Specifically, based on the results of gene expression analysis immediately after cortical injury (Day 0) and 7 days thereafter, candidate factors whose gene expression was high 7 days after cortical injury were extracted based on the following criteria (a1) and (b) or (a2) and (b):
(a1) membrane proteins & secretory factors and secretory factors: Log Fold-change (FC) of 2.0 or higher between Day 0 vs. Day 7; or
(a2) membrane proteins: Log Fold-change (FC) of 2.5 or higher between Day 0 vs. Day 7; and
   (b) p value ≤ 0.05 and CPM value of 10 or higher on Day 7.

### (Result)

The extracted factors are shown in the following Table 9.

### [Table 9]

**Table 9: Factors extracted in Example 3**

| Membrane proteins & secretory factors | | Secretory factors | | Membrane proteins | |
|---|---|---|---|---|---|
| Cpm ≥ 10 and Log FC >_ 2 | | Cpm ≥ 10 and Log FC ≥ 2 | | Cpm ≥ 10 and Log FC ≥ 2.5 | |
| 1 | Csf2ra | 1 | Apoc2 | 1 | Lilrb4 |
| 2 | Lag3 | 2 | Cd5l | 2 | Clec7a |
| 3 | Cxcl16 | 3 | Cst7 | 3 | Tlr2 |
| 4 | Cd44 | 4 | Cxcl 10 | 4 | Gpnmb |
| 5 | Tnfrsf1a | 5 | Lgals3 | 5 | C3ar1 |
| 6 | Csf1 | 6 | A2m | 6 | Ecel1 |
| 7 | Tnfrsf1b | 7 | Apoc1 | 7 | Tmem106a |
| | | 8 | Cxcl5 | 8 | Ms4a7 |
| | | 9 | C4b | 9 | Cd48 |
| | | 10 | Klk6 | 10 | Cd52 |
| | | 11 | Lgals3bp | 11 | Ifi30 |
| | | 12 | Ly86 | 12 | Cd180 |
| | | 13 | Isg 15 | 13 | Fcgr2b |
| | | 14 | C1qa | 14 | Hvcn 1 |
| | | 15 | C1qb | 15 | Mpeg1 |
| | | 16 | Ctsd | 16 | Bst2 |
| | | 17 | C1qc | 17 | Rtp4 |
| | | 18 | Cpxm1 | 18 | Trem2 |
| | | 19 | Grn | 19 | Cd68 |
| | | 20 | Lgals9 | 20 | Hk2 |
| | | 21 | Sparc | 21 | Kcne1l |
| | | 22 | Apod | 22 | Abca1 |
| | | 23 | Olfml3 | 23 | Icam 1 |
| | | 24 | Serping1 | 24 | P2ry6 |
| | | 25 | Col18a1 | 25 | Tbxas1 |
| | | | | 26 | Slc37a2 |
| | | | | 27 | Hmox 1 |
| | | | | 28 | Epsti1 |
| | | | | 29 | Slc15a3 |
| | | | | 30 | Cd74 |
| | | | | 31 | Cyba |
| | | | | 32 | Pld4 |
| | | | | 33 | Tyrobp |
| | | | | 34 | Cd84 |
| | | | | 35 | Emr 1 |
| | | | | 36 | Csf3r |

### <Example 4: Effects of promoting extension of neuronal axons by each factor>

### (Purpose)

A recombinant protein of each factor is prepared using culture cells, and effects of promoting extension of axons on cortical neurons are examined *in vitro.*

### (Method)

### (1) Preparation of culture supernatant containing a recombinant protein

In order to perform functional analysis of a total of 14 factors identified in Example 2, recombinant proteins were prepared by the following method. The mouse-derived full-length sequence of each factor was cloned and incorporated into an expression vector, and a plasmid was extracted and transfected to HEK293T cells. Then, recovery culture was performed for 1 or 2 days, culture was performed in a medium containing a transfection medium and DMEM/F12 medium (DMEM/F12 with L-glutamine, penicillin/streptomycin, N2-supplement, B27-supplement) mixed at 1:1 for one day, and further culture was performed for one day in DMEM/F12 medium (DMEM/F12 with L-glutamine, penicillin/streptomycin, N2-supplement, B27-supplement medium). The culture supernatant was filtered through a 0.22 µm filter and then cryopreserved.

### (2) Investigation of effects of promoting extension of neuronal axons

The cerebral cortex tissue was isolated from the brain of mice (C57BL/6NCrSlc, Shimizu Laboratory Supplies Co., Ltd.) on postnatal day 14.5, and only live cells were separated using a cell sorter. Then, the obtained cells were seeded on a plate coated with poly-L-lysine and LN511 (0.5 µg/cm²) at the cell density of 5,000 cells/cm². Culture was performed in a medium containing the DMEM/F12 medium (DMEM/F12 with L-glutamine, penicillin/streptomycin, N2-supplement, B27-supplement) mixed with each culture supernatant obtained in (1) described above at 1:1 for 2 days, immunostaining was performed with anti-TAU antibody (MAB3420, Merck Millipore), and neuronal axons were visualized to quantify the length of neuronal axons.

### (Result)

Fig. 6 shows the results of various recombinant proteins. An increased length of TAU-positive neuronal axons was observed for CCL12, CD52, CST7, LYZ2, MPEG1, RAC2, A2M, GM11428, GM14295, KLK6, SERPINA3N, and SPP1, compared with control (Fig. 6).

In the following examples, LYZ2 was selected as an example from the factors described above and subjected to further functional analysis. It is considered that effects of LYZ2 found in the following examples are to be obtained with other factors.

### <Example 5: Effects of mouse LYZ2 recombinant protein for promoting extension of neuronal axons>

### (Purpose)

LYZ2 is selected as an example from the factors described above, and functions of mouse LYZ2 (mLYZ2) are analyzed using the mLYZ2 recombinant protein expressed and purified using the Brevibacillus expression system and a commercially available mLYZ2 recombinant protein.

### (Method and result)

### (1) Preparation of mLYZ2 recombinant protein using Brevibacillus expression system

The mLYZ2 recombinant protein expressed with the use of the Brevibacillus expression system was subjected to His tag purification and protein concentration. The purified and concentrated samples were subjected to SDS-PAGE and CBB staining. The results showed that the mLYZ2 recombinant protein was obtained (Fig. 7). In addition, the mLYZ protein was detected by Western blotting using the anti-LYZ2 antibody (LS-C295234-100, LSBio) (Fig. 8).

### (2) Functional analysis of mLYZ2 recombinant protein prepared using Brevibacillus expression system

The cerebral cortex tissue was isolated from the brain of mice (C57BL/6NCrSlc, Shimizu Laboratory Supplies Co., Ltd.) on postnatal Day 14.5, and only live cells were separated using a cell sorter. Then, the obtained cells were seeded on a plate coated with poly-L-lysine and LN511-E8 fragment (0.5 µg/cm²) at the cell density of 2,000 cells/cm². Culture was performed in a neurobasal medium supplemented with the mLYZ2 recombinant protein prepared in (1) described above at the final concentration of 10% (Neurobasal medium with 2 mM L-glutamine, 1× penicillin/streptomycin, 1× B27-supplement, 10 µM Y-27632) for 3 days, immunostaining was performed with anti-TAU antibody (MAB3420, Merck Millipore), and the length of neuronal axons was quantified. As a result, it was observed that the mLYZ2 recombinant protein has an effect of promoting extension of a neuronal axon (Fig. 9).

### (3) Functional analysis using a commercially available mLYZ2 recombinant protein

Using a commercially available mLYZ2 recombinant protein (MBS1022200, MyBiosource), the activity of extending an axon was further investigated. The cortical tissue was isolated from mice on postnatal day 14.5, and, following the enzymatic treatment, the cells were seeded on a plate coated with poly-L-lysine and LN511-E8 fragment (0.5 µg/cm²). After culturing in a medium supplemented with the commercially available mLYZ2 recombinant protein at 0 ng/mL, 1 ng/mL, 10 ng/mL, or 100 ng/mL (DMEM/F12 supplemented with L-glutamine (2 mM), B27-supplement (1×), N2-supplement (1×), penicillin/streptomycin (1×)) for 6 days, immunostaining with anti-TAU antibody and evaluation were carried out. The results demonstrated that the neuronal axons were extended (Fig. 10) and the neuronal axon collaterals were increased (Fig. 11) when the commercially available mLYZ2 was used.

### <Example 6: Effects of human LYZ recombinant protein for extending a neuronal axon>

### (Purpose)

Effects of the human LYZ (hLYZ) recombinant protein on human neurons (human cerebral cortex neurons and human dopamine neurons) are investigated.

### (Method and result)

### (1) Cerebral cortex organoid derived from human ES cells

As a human LYZ (hLYZ) recombinant protein, the commercially available hLYZ (ab158839, Abcam) was used. The cerebral cortex organoid derived from human ES cells prepared in accordance with the method described in a literature (Sakaguchi, H., et al., Stem Cell Reports., 2019, 13 (3): 458-473) was dispersed by enzymatic treatment, and then, the cells were cultured in a medium supplemented with hLYZ at 0 ng/mL, 1 ng/mL, 10 ng/mL, 100 ng/mL, or 1,000 ng/mL (DMEM/F 12 supplemented with L-glutamine (2 mM), B27-supplement (1×), N2-supplement (1×), Penicillin/Streptomycin (1×), Y-27632 (10 µM)) for 3 days. Then, the TAU-positive neuronal axons were visualized via immunostaining using the anti-TAU antibody, and the length of neuronal axons was quantified. The results demonstrated that TAU-positive neuronal axons of the neurons derived from the cerebral cortex organoid derived from human ES cells were extended by hLYZ (Fig. 12).

### (2) Dopamine neurons derived from human ES cells

The dopamine neurons derived from human ES cells which were subjected to induction of differentiation in accordance with the method described in the literature (Kriks S., et al., Nature, 2011, 480 (7378): 547-51) and dispersion by enzymatic treatment were cultured in a medium supplemented with hLYZ at 0 ng/mL, 1 ng/mL, 10 ng/mL, 100 ng/mL, or 1,000 ng/mL (Neurobasal medium supplemented with GlutaMAX-I (1%), B27-supplement (1×), Y-27632 (10 µM)) for 3 days. Then, TAU-positive neuronal axons were visualized by immunostaining using anti-TAU antibody, and the length of neuronal axons was quantified. The results demonstrated that TAU-positive neuronal axons of the dopamine neurons derived from human ES cells were extended by hLYZ (Fig. 13).

### <Example 7: Effects on the cell number of neurons>

### (Purpose)

Effects of the commercially available mouse LYZ2 recombinant protein (mLYZ2) and the human LYZ recombinant protein (hLYZ) on the total cell number of TAU-positive neurons are examined.

### (Method and result)

### (1) Effects of mLYZ2 on mouse cerebral cortex neurons

The cerebral cortex cells of Black6/N mice on Day 14 of gestation were cultured in a medium supplemented with the commercially available mLYZ2 recombinant protein (MBS1022200, MyBiosource) at 0 ng/mL, 1 ng/mL, or 10 ng/mL (DMEM/F 12 supplemented with L-glutamine (2 mM), B27-supplement (1×), N2-supplement (1×), penicillin/streptomycin (1×)) for 6 days. Then, the length of neuronal axons of TAU-positive neurons and the total cell number thereof were quantified by immunostaining using anti-TAU antibody. The results demonstrated that the total cell number of TAU-positive neurons was increased (Fig. 14). This result indicated that mLYZ2 has an effect of suppressing neuronal cell death.

### (2) Effects of hLYZ on human neurons

Cells obtained by the enzymatic treatment of the cerebral cortex organoid derived from human iPS cells on Day 46 from induction of differentiation (prepared in accordance with the method described in Kriks S., et al., Nature, 2011, 480 (7378): 547-51) were cultured in a medium supplemented with the commercially available hLYZ (ab158839, Abcam) at 0 ng/ml, 1 ng/ml, 10 ng/ml, or 100 ng/ml (DMEM/F12 supplemented with L-glutamine (2 mM), B27-supplement (1×), N2-supplement (1×), penicillin/streptomycin (1×), Y-27632 (10 µM))) for 13 days. Then, the length of neuronal axons of TAU-positive neurons and the total cell number thereof were quantified by immunostaining using anti-TAU antibody. The results demonstrated that the total cell number of neurons increased (Fig. 15).

This result indicated that hLYZ has an effect of suppressing neuronal cell death.

### <Example 8: Effects of mouse LYZ2 recombinant protein on transplanted cells>

### (Purpose)

Simultaneously with the transplantation of a population of cells separated from the embryonic mouse brain into the brain of adult mice, the mouse LYZ2 recombinant protein (mLYZ2) is administered. Effects of mLYZ2 on survival of transplanted cells and differentiation into neurons are examined.

### (Method and result)

The ventral mesencephalic tissue including the dopamine neural progenitor cells was isolated from the brain of the embryonic mouse (C57BL/6-Tg (CAG-EGFP), Shimizu Laboratory Supplies Co., Ltd.) on Day 11 of gestation. After the tissue was subjected to enzymatic treatment, a cell suspension containing 100,000 cells was prepared using PBS containing Y-27632 at the final concentration of 10 µM. This cell suspension was supplemented with mLYZ2 (20 ng) and transplanted into the striatum of 12-week-old Black6/N mice (male). A group of mice to which the cell suspension not supplemented with mLYZ2 was transplanted were designated as a control group.

One month after transplantation, the mice were subjected to perfusion fixation, and frozen brain sections were prepared. Thereafter, immunostaining was performed using anti-TH antibody (AB152, Merck Millipore) which labels dopamine-positive cells, and the number of dopamine-positive cells included in the transplant were counted to calculate the cell density. The results are shown in Fig. 16.

In the experimental group to which the transplanted cells were transplanted in combination with mLYZ2 (Fig. 16, Lyz), the density of dopamine-positive cells was considerably increased, compared with the control group (Fig. 16, Ctrl) (Fig. 16).

Accordingly, it was demonstrated that mLYZ2 can be used as an agent for assisting cell transplantation.

### <Example 9: Effects of the human Lyz recombinant protein on transplanted cells>

### (Purpose)

Dopamine neuron progenitor cells derived from human ES cells are cultured in the presence of the human Lyz recombinant protein for a certain period of time and transplanted into the brain of an adult mouse. Effects of human Lyz on survival of the transplanted cells are examined.

### (Method and result)

Dopamine neuron progenitor cells were prepared from human ES cells (Kh-ES1) in accordance with the method described in the literature (Doi D, et al., Stem Cell Reports, 2014, 6; 2 (3): 337-50). Cell culture was performed with the addition of the human Lyz recombinant protein (Recombinant Human Lysozyme protein; abcam/ab158839) at 100 ng/ml to the culture medium from Day 18 of culture. The dopamine neuron progenitor cells were subjected to enzymatic treatment on Day 29 of culture, and a cell suspension containing 200,000 cells was prepared using PBS containing Y-27632 at the final concentration of 10 µM. In the present example, Lyz was not additionally added to the cell suspension. The cell suspension was transplanted to the striatum of 16-week-old immunodeficient mice (SCID mice; Shimizu Laboratory Supplies Co., Ltd., C.B-17/lcrHsd-Prkdcscid) (Lyz group). A group of mice subjected to the transplantation of the dopaminergic neural progenitor cells cultured without the addition of Lyz to the culture medium were designated as a control group (Ctrl).

Three months after transplantation, the mice were subjected to perfusion fixation, frozen brain sections were prepared, and the transplanted cells were labeled by antibody staining to visualize the transplant. In antibody staining, the mouse anti-hNCAM antibody (Santa Cruz sc-106) was used at the dilution ratio of 1:500. Fig. 17 shows the results of comparison of the transplant size between Lyz group and control group.

As shown in Fig. 17, the dopamine neuron progenitor cells cultured in the presence of Lyz (Lyz group) exhibited significantly increased transplant size, compared with the cells cultured in the absence of Lyz (control group). The results demonstrated that cell survival is significantly promoted in the transplantation of cells pretreated with Lyz.

### <Example 10: Evaluation of effects of promoting extension of neuronal axons and effects of suppressing cell death by further candidate factors>

### (Purpose)

Effects of promoting extension of neuronal axons and effects of suppressing cell death are evaluated for 7 candidate factors: Lysozyme (LYZ), Secreted phosphoprotein 1 (SPP1 or Osteopontin), Progranulin (PGrn), Cathepsin D (Ctsd), Cathepsin S (Ctss), Apolipoprotein D (Apod), and Sparc.

### (Method and result)

### (1) Candidate factors

As each candidate factor, a human recombinant protein was used. Specifically, the Recombinant Human Lysozyme protein (abcam, ab158839) was used as the human LYZ recombinant protein, the Recombinant Human Osteopontin (OPN) Protein (R & D Systems, 1433-OP-050/CF) was used as the human SPP1 recombinant protein, the Recombinant Human Progranulin protein (R & D Systems, 2420-PG-050) was used as the human PGrn recombinant protein, the Recombinant Human Cathepsin D Protein (R & D Systems, 1014-AS-010) was used as the human Ctsd recombinant protein, the Recombinant Human Cathepsin S Protein (R & D Systems, 1183-CY-010) was used as the human Ctss recombinant protein, the Recombinant Human Apolipoprotein D Protein (Novus Biologicals, NBP1-99548) was used as the human Apod recombinant protein, and the Recombinant Human SPARC Protein (R & D Systems, 941-SP-050) was used as the human Sparc recombinant protein.

### (2) Evaluation of effects of promoting extension of neuronal axons

The cerebral cortex organoid derived from human iPS cells (S2WCB3) on Day 42 to Day 70 after induction of differentiation (prepared in accordance with the method described in Kriks S., et al., Nature, 2011, 480 (7378): 547-51) was dissociated using the Neuron Dissociation Solutions (FUJIFILM), and resulting cells were seeded at the cell density of 20,000 cells/cm² on a 96-well plate coated with laminin (LM511-E8) on its surface. Culture was performed in a medium (DMEM/F12 supplemented with L-glutamine (2 mM), B27-supplement (1×), N2-supplement (1×), penicillin/streptomycin (1×), Y-27632 (10 µM)) for 7 days (from Day 0 to Day 7).

On Day 1 of culture, which is one day after seeding cells, each candidate factor of (1) described above was added to the medium, and the culture was continued in the presence of each candidate factor up to Day 7. Each factor was diluted with the medium to the final concentration per well (200 µl) shown in Fig. 18 to Fig. 21. During the culture for 7 days, temporal changes in the axonal length of each cell were measured by live cell imaging using IncuCyte.

The results are shown in Fig. 18 and Fig. 19. Fig. 18 shows temporal changes in the length of axons when Lyz was added to the medium. When Lyz was added at 100 ng/mL, 250 ng/mL, and 500 ng/mL to the medium, it was shown that effects of extending axons were greater when a larger amount of Lyz was added (Fig. 18B). Fig. 19 shows the results of measuring the length of axons on Day 7 of culture in the presence of each factor. It was shown that the length of axons increased in the presence of Lyz. In addition, a tendency that the length of axons increased also in the presence of PGrn, Spp1, or Sparc was observed.

### (3) Evaluation of cell viability

After the completion of live cell imaging on Day 7 of culturing in (2) described above, the cell viability was evaluated by the Alamar Blue assay. In the measurement method, the cerebral cortex organoid on Day 47 to Day 70 of induction of differentiation was dispersed and seeded at the cell density of 40,000 cells/cm² on a dish coated with laminin (LM511-E8). The composition of the medium used here was DMEM/F12 supplemented with L-glutamine (2 mM), B27-supplement (1×), N2-supplement (1×), Penicillin/Streptomycin (1×), and Y-27632 (10 µM). Culturing was performed for 24 hours after seeding, and various proteins were added, and immediately thereafter, 0.1 mM H₂O₂ was added, and the cell viability after 24 hours was evaluated. The composition of the medium used here was DMEM/F 12 supplemented with L-glutamine (2 mM), B27-supplement (1×), N2-supplement (1×), and Penicillin/Streptomycin (1×). The Alamar Blue assay was performed with the use of alamarBlue^{®} Cell Viability Reagent (Thermo Fisher Scientific, DAL1025) in accordance with the method attached to the reagent. As the control groups, cells without the addition of candidate factors (Fig. 20, Ctrl mean) and cells artificially injured by treatment with a surfactant (1% Triton) (Fig. 20, 1% Triton mean) were also evaluated.

Fig. 20 shows the results of evaluation of the cell viability. In all of the candidate factors used, a tendency that cell viability increased was observed, compared with the control groups. In particular, the tendency was high in Lyz and PGrn.

### (4) Evaluation of cell viability under cytotoxic (H₂O₂) conditions

In the same manner as in (2) described above, the cells dissociated from the cerebral cortex organoid derived from human iPS cells on Day 42 to Day 70 of induction of differentiation were seeded at the cell density of 40,000 cells/cm² on a 96-well plate. After culturing was performed for 24 hours from the seeding, 0.1 mM H₂O₂ and each candidate factor indicated in (1) described above were added to the medium. After culturing was performed in the presence of 0.1 mM H₂O₂ and each candidate factor for 24 hours, the cell viability was measured by the Alamar Blue assay in the same manner as in (3) described above.

Fig. 21 shows the results. The results shown in Fig. 21 demonstrated that the cell viability under cytotoxic (H₂O₂) condition was increased by PGrn. A tendency that the cell viability increased under the cytotoxic (H₂O₂) condition was observed also when Spp1, Sparc, or Lyz was used.

All publications, patents, and patent applications cited herein are incorporated herein by reference in their entirety.

## Claims

1. An agent for activating a neuron comprising a protein, an active fragment thereof or a nucleic acid encoding the protein or active fragment thereof,
wherein the protein is selected from the group consisting of Lysozyme (LYZ), Secreted phosphoprotein 1 (SPP1), Progranulin (PGrn), Cathepsin D (Ctsd), Cathepsin S (Ctss), Apolipoprotein D (Apod), Sparc, CD52, Macrophage expressed gene 1 (MPEG1), C-C motif chemokine ligand 2 (CCL2), Cystatin F (CST7), Ras family small GTPase 2 (RAC2), Serpin family A member 3 (SERPINA3), Anosmin 1 (ANOS 1), Kallikrein-related peptidase 6 (KLK6), Alpha-2-macroglobulin (A2M), GM14295, Galectin 3-binding protein (LGALS3BP), Apolipoprotein C1 (APOC1), and orthologs thereof.

2. The agent according to claim 1, wherein the neuron is a brain neuron.

3. The agent according to claim 1 or 2, wherein said activating a neuron is a promotion of extension of an axon, an axon collateral, and/or a dendrite.

4. The agent according to claim 1 or 2, wherein said activating a neuron is a suppression of neuronal degeneration and/or neuronal cell death.

5. An agent for assisting cell transplantation comprising the agent according to any one of claims 1 to 4.

6. An agent for treating or preventing a neural disease comprising the agent according to any one of claims 1 to 4.

7. The agent according to claim 6, wherein the disease is selected from the group consisting of neurodegenerative disease, cerebral tumor, multiple sclerosis, epilepsia, head trauma, cerebral infarction, cerebral stroke, and ischemia.

8. The agent according to claim 7, wherein the neurodegenerative disease is selected from the group consisting of Parkinson's disease, Alzheimer's disease, Huntington's disease, amyotrophic lateral sclerosis, dementia with Lewy bodies, spinocerebellar degeneration, progressive supranuclear palsy, and corticobasal degeneration.
